(19)

Europäisches
Patentamt
European
Patent Office
Office européen
des brevets

(11) **EP 3 636 269 A1**

(12) **EUROPEAN PATENT APPLICATION**
published in accordance with Art. 153(4) EPC

(43) Date of publication:
**15.04.2020 Bulletin 2020/16**

(21) Application number: **18793787.5**

(22) Date of filing: **27.04.2018**

(51) Int Cl.:
*A61K 31/737* (2006.01)     *A61K 39/395* (2006.01)
*A61P 35/00* (2006.01)

(86) International application number:
**PCT/CN2018/084769**

(87) International publication number:
**WO 2018/201981 (08.11.2018 Gazette 2018/45)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.05.2017   US 201762492525 P
16.01.2018   CN 201810051881**

(71) Applicant: **China Medical University
Taichung City 404 (TW)**

(72) Inventors:
• **SHYU, Woei-Cherng
Taichung City 404
Taiwan (TW)**

• **CHEN, San-Yuan
Hsinchu City 300
Taiwan (TW)**
• **CHIANG, Chih-Sheng
Taichung City 427
Taiwan (TW)**
• **HSIEH, Chia-Hung
Taichung City 404
Taiwan (TW)**
• **LIN, Yu-Jung
Taichung City 404
Taiwan (TW)**
• **TSAI, Chang-hai
Taichung City 404
Taiwan (TW)**

(74) Representative: **D Young & Co LLP
120 Holborn
London EC1N 2DY (GB)**

(54) **IMMUNOMAGNETIC COMPOSITION, PREPARATION METHOD AND USE THEREOF, AND KIT FOR TREATING CANCER**

(57)     An immunomagnetic composition, a preparation method and a use thereof, and a kit for treating a cancer. The immunomagnetic composition comprises a core layer, a shell layer and an outer layer. The shell layer is composed of a composite and encapsulates the core layer. The composite is formed by the combination of fucoidan, oxidized dextran, and a plurality of superparamagnetic iron oxide nanoparticles by hydrophobic interaction. The outer layer comprises at least one antibody.

Fig. 1

EP 3 636 269 A1

**Description**

**TECHNICAL FIELD**

The present disclosure relates to a composition and a preparation method thereof. More particularly, the present disclosure relates to an immunomagnetic composition, a preparation method and use thereof characterized by special physical form, and a kit for treating cancer.

**DESCRIPTION OF RELATED ART**

Cancer, also known as malignancy, is a state of abnormal proliferation of cells, and these proliferating cells may invade other parts of the body as a disease caused by a malfunction in the control of cell division and proliferation. The number of people suffering from cancer worldwide has a growing trend. Cancer is one of the top ten causes of death for the Chinese people and has been the top ten causes of death for twenty-seven consecutive years.

Conventional cancer treatments include surgery, radiation therapy, and chemotherapy. Immunotherapy is another method of treating cancer except the above methods. The immune system of the patient is activated in the immunotherapy by using tumor cells or tumor antigens to induce specific cellular and humoral immune responses for enhancing the anti-cancer ability of the patient, preventing the growth, spread, and recurrence of tumors, and achieving the purpose of removing or controlling tumors. The immune checkpoint is one of the most important types of immunotherapy. There have been over 50 clinical trials using immune checkpoint inhibitors combination therapy since 2015. However, the immune checkpoint inhibitor will shut down the feedback mechanism of the human immune system, so that cytotoxic T cells ($CD8^+$ T cells) not only attack cancer cells, but also generate autoimmune reactions such as skin ulcers and gastrointestinal ulcers.

Increasing the immune cells that are specific to tumors in the body is also considered a very promising part of cancer treatment. In most of the current technologies, the immune cells are obtained from tumors of patients and then cultured *in vitro.* Micron-sized structures (such as microbeads) are used to mimic antigen presenting cell (APCs) for T cell proliferation and training. Finally, the trained immune cells are transferred back into the patient's body to kill cancer cells. However, the above method is time consuming and consumable, and the cancer cells in the patient's body are prone to mutation, so that the returned immune cells lose their effect. On the other hand, microbeads used for proliferation can only be used for *in vitro* culture due to their large size and cannot be circulated into the target area via human blood. In addition to surface immobilized antibodies or encapsulated active ingredients in this type of carriers, the carrier-forming material is usually an excipient. The excipient does not contribute much to the therapeutic effect and will limit the administration dose, becoming an inherent defect in this type of carriers.

**DISCLOSURE OF INVENTION**

The purpose of the present disclosure is to provide an immunomagnetic composition which can be used for preparing an anticancer drug, a preparation method thereof, use thereof, and a kit for treating cancer.

According to one aspect of the present disclosure, an immunomagnetic composition is provided. The immunomagnetic composition includes a core, a shell and an outer layer. The shell is formed by a complex, and the complex is fabricated by a combination of fucoidan, oxidized dextran, and a plurality of superparamagnetic iron oxide nanoparticles via a hydrophobic interaction. The core is encapsulated in the shell. The outer layer includes at least one antibody immobilized onto outside of the shell to form the outer layer, wherein the antibody is an immune checkpoint inhibitor and/or a T cell expansion antibody.

In one example, the immunomagnetic composition can be a sphere with a particle size ranging from 80 nm to 350 nm.

In one example, the fucoidan can be extracted from Undaria pinnatifida, Macrocystis pyrifera, or Fucus vesiculosus.

In one example, the oxidized dextran can have an aldehyde group. In one example, the oxidized dextran can be prepared from a dextran with a molecular mass ranging from 5 kDa to 270 kDa.

In one example, the immune checkpoint inhibitor can be selected from the group consisting of a PD-L1 antibody, a PD-1 antibody, a CTLA-4 antibody and a TIM-3 antibody. In one example, the T cell expansion antibody can be selected from the group consisting of a CD3 antibody, a CD28 antibody and a 4-1BB antibody.

In one example, the core can further include an active substance.

Therefore, the immunomagnetic composition of the present disclosure utilizes the fucoidan with anticancer activity, the oxidized dextran, and the superparamagnetic iron oxide nanoparticles as the carrier component, thereby forming a nanoscale structure having an antibody on the outer layer and coating the active substance in the core layer. The size and surface charge of the immunomagnetic composition are suitable, which can increase the circulation time in the body and penetrate into the tumor for enhancing the effect of the fucoidan on the tumor. The antibody immobilized onto the outer layer can be the immunosense checkpoint inhibitor and/or the T cell expansion agent, so that the immunomagnetic composition of the present disclosure can also be the immune checkpoint inhibitor and/or the T cell expansion agent in addition to the anti-cancer function of its own material for significantly improving the tumor microenvironment. Moreover, the immunomagnetic composition of the present disclosure can markedly improve the anti-cancer effect of the immunotherapy with the same antibody alone, and can achieve better tumor inhibition with less antibody dosage. The fabricated immunomagnetic composition can be lyophilized to form the powdery crystals and stored under aseptic conditions for a long period of time. The lyophilized powdery crystal can be rapidly re-dispersed in the solvent when needed, showing its convenience and stability.

According to another aspect of the present disclosure, a preparation method of an immunomagnetic composition is provided. The preparation method of the immunomagnetic composition includes providing a hydrophilic phase solution, providing an organic phase solution, performing an emulsification, removing the organic solvent in the emulsion and performing an antibody immobilization. In the step of providing the hydrophilic phase solution, the hydrophilic phase solution includes a fucoidan and an oxidized dextran. In the step of providing the organic phase solution, the organic phase solution includes an organic solvent and a plurality of superparamagnetic iron oxide nanoparticles. In the step of performing an emulsification, the hydrophilic phase solution and the organic phase solution are mixed to form an emulsion. The organic solvent in the emulsion is removed to form a magnetic fucoidan nanoparticle. In the step of the antibody immobilization, at least one antibody is mixed with the magnetic fucoidan nanoparticle to form the immunomagnetic composition, and the least one antibody is an immune checkpoint inhibitor and/or a T cell expansion antibody.

In one example, the immune checkpoint inhibitor can be selected from the group consisting of a PD-L1 antibody, a PD-1 antibody, a CTLA-4 antibody and a TIM-3 antibody. In one example, the T cell expansion antibody can be selected from the group consisting of a CD3 antibody, a CD28 antibody and a 4-1BB antibody.

In one example, a weight ratio of the fucoidan and the oxidized dextran can be 1: 0.1 to 1: 4.

In one example, the oxidized dextran can have an aldehyde group. In one example, the oxidized dextran can be prepared from a dextran with a molecular mass ranging from 5 kDa to 270 kDa.

In one example, the organic solvent can be methanol, dichloromethane or chloroform.

Therefore, the preparation method of the immunomagnetic composition of the present disclosure is different from the complicated manufacturing process of other target carriers. The preparation method of the immunomagnetic composition of the present disclosure does not require the use of surfactant to stabilize the structure, and the acquisition and production of materials is also quite easy.

According to yet another aspect of the present disclosure, a use of the immunomagnetic composition according to the aforementioned aspect in manufacture of a pharmaceutical composition for treating cancer is provided.

In one example, the pharmaceutical composition for treating cancer can be a pharmaceutical composition for inhibiting a cancer cell proliferation. In one example, the pharmaceutical composition for treating cancer can be a pharmaceutical composition for reducing a cancer cell metastasis. In one example, the pharmaceutical composition for treating cancer can be a pharmaceutical composition for triggering a tumor immune response.

According to yet another aspect of the present disclosure, a kit for treating cancer is provided. The kit includes the immunomagnetic composition according to the aforementioned aspect and a magnetic field generator.

Therefore, the kit for treating cancer includes the immunomagnetic composition and the magnetic field generator, wherein the magnetic field generator can be the auxiliary tool to generate the magnetic field for the magnetic navigation. Thus, the immunomagnetic composition of the present disclosure in the kit for treating cancer can be accumulated in the affected part to achieve the effect of local magnification treatment and avoid systemic immune response. Therefore, the kit for treating cancer of the present disclosure simultaneously has the physical target function and biological target function, which is helpful for the immunotherapy with the chemotherapy or the combination therapy of the immunotherapy. The dose of the antibody in the kit for treating cancer of the present disclosure is only one percent of the dose of pure antibody administered in general, and the kit for treating cancer is able to exhibit more excellent tumor inhibiting ability and extend the half-life by more than 2 times.

These and other features, aspects, and advantages of the present invention will become better understood with reference to the following description and appended claims. It is to be understood that both the foregoing general description and the following detailed description are by examples, and are intended to provide further explanation of the invention as claimed.

## BRIEF DESCRIPTION OF THE DRAWINGS

The present disclosure can be more fully understood by reading the following detailed description of the embodiments, with reference made to the accompanying drawings as follows:

Fig. 1 is a structural schematic view showing an immunomagnetic composition according to the present disclosure;

Fig. 2 is a flow chart showing a preparation method of the immunomagnetic composition according to the present disclosure;

Figs. 3A to 3F show structural analysis results of a magnetic fucoidan nanoparticle;

Figs. 4A to 4C show structural analysis results of a magnetic nanoparticle IO@Fu;

Figs. 5A and 5B show structural analysis results of a magnetic nanoparticle IO@Dex;

Figs. 6A to 6C show stability analysis results of the magnetic fucoidan nanoparticle;

Figs. 7A and 7B are transmission electron micrographs of the magnetic fucoidan nanoparticle before and after lyophilized;

Fig. 8 shows structural analysis results of an immunomagnetic composition according to one example of the present disclosure;

Fig. 9 shows X-ray photoelectron spectroscopy analysis results of the immunomagnetic composition according to one example of the present disclosure;

Figs. 10A and 10B show structural analysis results of an immunomagnetic composition according to another example of the present disclosure;

Figs. 11A to 11F show targeting ability analysis and cell association ability analysis results of the immunomagnetic composition of the present disclosure;

Figs. 12A to 12D show cell association ability analysis results of the immunomagnetic composition of the present disclosure;

Figs. 13A to 13C show analysis results of nanomedicine accumulation in tumor of the kit for treating cancer of the present disclosure;

Fig. 13D are hematoxylin and eosin stain with Prussian blue stain photographs showing treatment effect of the immunomagnetic composition and the kit for treating cancer of the present disclosure;

Figs. 14A to 14E show analysis results of cancer cell proliferation inhibiting capacity and anti-metastasis capacity in a breast cancer metastasis mouse model of the immunomagnetic composition and the kit for treating cancer of the present disclosure;

Figs. 15A to 15C show analysis results of cancer cell proliferation inhibiting capacity in a colorectal cancer mouse model of the immunomagnetic composition and the kit for treating cancer of the present disclosure;

Figs. 16A to 16I show analysis results of changes of tumor-infiltrating lymphocytes and cytokines in tumor microenvironment after administering the immunomagnetic composition and the kit for treating cancer of the present disclosure;

Figs. 17A and 17B show reaction sites analysis results of the immunomagnetic composition and the kit for treating cancer of the present disclosure;

Figs. 17C and 17D show TUNEL analysis results of the immunomagnetic composition and the kit for treating cancer of the present disclosure;

Figs. 18A to 18E show analysis results of infiltration degree of $CD4^+$ T cells and $CD8^+$ T cells of mice after administering the immunomagnetic composition and the kit for treating cancer of the present disclosure;

Figs. 19A to 19D show blood biochemical analysis results of the mice after administering the immunomagnetic composition and the kit for treating cancer of the present disclosure; and

Fig. 20 shows histology pictures of the mice after administering the immunomagnetic composition and the kit for treating cancer of the present disclosure.

## DETAILED DESCRIPTION

A novel immunomagnetic composition is provided in the content of the specification. The immunomagnetic composition is fabricated by combining a fucoidan, an oxidized dextran, and a plurality of superparamagnetic iron oxide nanoparticles via a hydrophobic interaction and then immobilizing antibody. The fabricated immunomagnetic composition can markedly improve an anti-cancer effect of immunotherapy with the same antibody alone and can achieve better tumor inhibition with less antibody dosage. In addition, a kit for treating cancer thereof including the immunomagnetic composition and a magnetic field generator is provided for enhancing the anti-cancer effect of the immunomagnetic composition of the present disclosure. The accompanied effects of the immunomagnetic composition and the kit for treating cancer disclosed in the experiments and the examples for demonstrating the effect and the mechanism of the immunomagnetic composition and the kit for treating cancer in the immunotherapy through a breast cancer metastasis mouse model and a colorectal cancer mouse model.

The following are descriptions of the specific terms used in the specification:

The term "fucoidan" is a water-soluble dietary fiber extracted from sticky and slippery components unique to brown algae. The fucoidan is rich in fucose, is a kind of natural polysaccharide with high biological safety, and has abilities of anti-oxidation, anti-coagulation, anti-thrombosis, anti-virus and anti-cancer.

The term "dextran" is a complex branched glucan (polysaccharide made of many glucose molecules) composed of chains of varying lengths from 3 Da to 2000 kDa. The straight chain consists of $\alpha$-1,6 glycosidic linkages between glucose molecules, while branches begin from $\alpha$-1,3 linkages. The term "oxidized dextran" is a surface-modified dextran, wherein the hydroxyl groups on the dextran are oxidized to aldehyde groups to obtain the oxidized dextran which can further immobilize antibody.

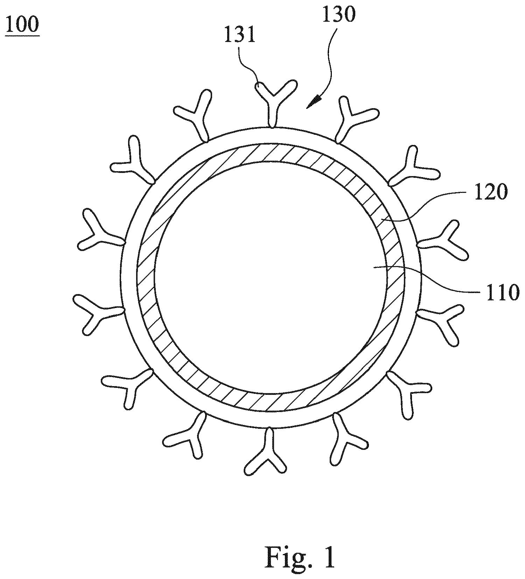

Please refer to Fig. 1, which is a structural schematic view showing an immunomagnetic composition 100 according to the present disclosure. As shown in Fig. 1, the immunomagnetic composition 100 includes a core 110, a shell 120 and an outer layer 130.

The core 110 can includes an active substance. The active substance can be a cytokine or an anti-cancer drug.

The shell 120 is formed by a complex, wherein the complex is fabricated by a combination of the fucoidan, the oxidized dextran, and a plurality of superparamagnetic iron oxide nanoparticles via a hydrophobic interaction. The core 110 is encapsulated in the shell 120. Preferably, the fucoidan used to form the complex of the shell 120 can be extracted from *Undaria pinnatifida*, *Macrocystis pyrifera*, or *Fucus vesiculosus.* The oxidized dextran used can have an aldehyde group and can be prepared from a dextran with a molecular mass ranging from 5 kDa to 270 kDa. The hydrophobic interaction between the fucoidan, the oxidized dextran, and the superparamagnetic iron oxide nanoparticles can be caused by methods such as an emulsification or a nano-precipitation method. However, the present disclosure is not limited thereto.

The outer layer 130 includes at least one antibody 131 immobilized onto outside of the shell 120 to form the outer layer 130. The antibody 131 is an immune checkpoint inhibitor and/or a T cell expansion antibody. Preferably, the immune checkpoint inhibitor can be selected from the group consisting of a PD-L1 antibody, a PD-1 antibody, a CTLA-4 antibody and a TIM-3 antibody. The T cell expansion antibody is selected from the group consisting of a CD3 antibody, a CD28 antibody and a 4-1BB antibody.

Preferably, the immunomagnetic composition 100 can be a sphere with a particle size ranging from 80 nm to 350 nm. In addition, the immunomagnetic composition 100 can be a hollow shape.

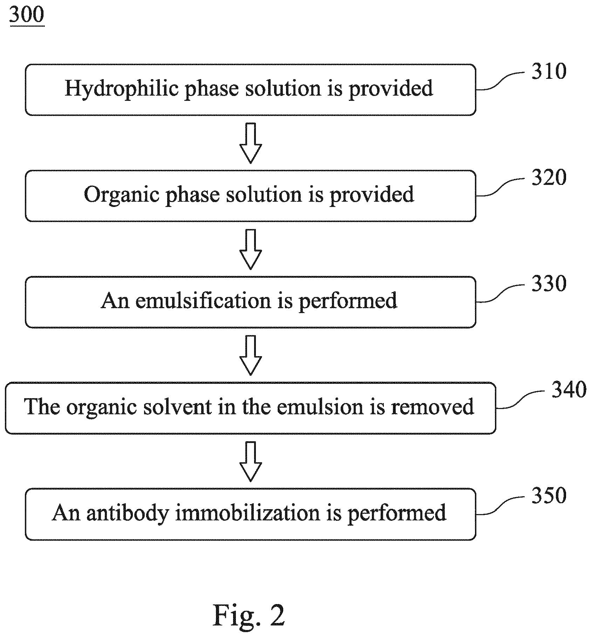

Please refer to Fig. 2, which is a flow chart showing a preparation method of the immunomagnetic composition 300 according to the present disclosure. The preparation method of the immunomagnetic composition 300 includes Step 310, Step 320, Step 330, Step 340 and Step 350.

In Step 310, a hydrophilic phase solution is provided. The hydrophilic phase solution includes the fucoidan and the oxidized dextran. The fucoidan can be extracted from *Undaria pinnatifida*, *Macrocystis pyrifera*, or *Fucus vesiculosus.* The oxidized dextran can be prepared from the dextran with the molecular mass ranging from 5 kDa to 270 kDa. The fucoidan and the oxidized dextran can be mixed in a weight ratio of 1: 0.1 to 1: 4.

In Step 320, an organic phase solution is provided. The organic phase solution includes an organic solvent and a plurality of superparamagnetic iron oxide nanoparticles. The organic solvent can be methane, dichloromethane or chloroform.

In Step 330, the emulsification is performed. The hydrophilic phase solution provided in step 310 and the organic phase solution provided in step 320 are mixed to form an emulsion.

In Step 340, the organic solvent in the emulsion can be removed by evaporation under reduced pressure to form a magnetic fucoidan nanoparticle.

In Step 350, an antibody immobilization is performed. At least one antibody is immobilized onto the magnetic fucoidan nanoparticle to form the immunomagnetic composition. The antibody used is the immune checkpoint inhibitor and/or the T cell expansion antibody. Preferably, the immune checkpoint inhibitor can be selected from the group consisting of the PD-L1 antibody, the PD-1 antibody, the CTLA-4 antibody and the TIM-3 antibody. The T cell expansion antibody can be selected from the group consisting of the CD3 antibody, the CD28 antibody and the 4-1BB antibody.

Accordingly, the immunomagnetic composition fabricated by the aforementioned preparation method can be used as the pharmaceutical composition for treating cancer. The pharmaceutical composition for treating cancer includes the immunomagnetic composition and a pharmaceutically acceptable carrier. The pharmaceutical composition for treating cancer can be used for inhibiting a proliferation of cancer cells, reducing a metastasis of cancer cells and triggering a tumor immune response. The immunomagnetic composition fabricated by the aforementioned preparation method is shown as the hollow shape, hence the active substance can be further encapsulated in the core of the immunomagnetic composition to enhance the anticancer effect of the pharmaceutical composition for treating cancer.

The immunomagnetic composition fabricated by the aforementioned preparation method can be cooperated with a magnetic field generator as the kit for treating cancer. The magnetic field generator can be a device which can generate a magnetic field, such as a magnet, a three-dimensional field magnet or a magnetic resonance imaging scanner. The magnetic field generated by the magnetic field generator can be used as an auxiliary tool for a magnetic navigation, and the immunomagnetic composition of the present disclosure can be accumulated in the affected part to achieve the effect of local treatment. Therefore, the dose of the antibody in the kit for treating cancer of the present disclosure is only one percent of the dose of pure antibody administered in general, and the kit for treating cancer is able to exhibit more excellent tumor inhibiting ability and extend the half-life by more than 2 times.

Reference will now be made in detail to the present embodiments of the present disclosure, examples of which are illustrated in the accompanying drawings. Wherever possible, the same reference numbers are used in the drawings and the description to refer to the same or like parts.

**<Experiments and Examples>**

**I. The immunomagnetic composition of the present disclosure and the preparation method thereof**

**1.1 Structure and stability analysis of the magnetic fucoidan nanoparticle**

To test the optimal preparation condition of the immunomagnetic composition, the magnetic fucoidan nanoparticle without immobilized antibody is fabricated in this experiment first. The structure of the magnetic fucoidan nanoparticle is analyzed by a scanning electron microscopy (SEM) and a transmission electron microscopy (TEM). The zeta potential (ZP) of the magnetic fucoidan nanoparticle, the particle size of the magnetic fucoidan nanoparticle and the stability of the magnetic fucoidan nanoparticle dispersed in distilled deionized water (DDW) and phosphate buffered saline (PBS) are analyzed by a particle analyzer (Delsa Nano C particle analyzer, BECKMAN COULTER).

Before fabricating the magnetic fucoidan nanoparticle, the superparamagnetic iron oxide nanoparticles and the oxidized dextran are prepared, respectively. The oxidized dextran used in this experiment has the aldehyde group for subsequent immobilizing the antibody. The superparamagnetic iron oxide nanoparticles (hereafter "IO") are modified and prepared by consulting the literature published by Shouheng Sun in 2004 (Shouheng Sun et al., Monodisperse MFe2O4 (M = Fe, Co, Mn) Nanoparticles. Journal of the American Chemical Society 2004, 126(1): 273-279). In brief, 2 mmol of $Fe(acac)_3$, 10 mmol of 1,2-hexadecanediol, 6 mmol of oleic acid, and 6 mmol of olecylamine are mixed in 20 ml of benzyl ether to refluxed at 100°C for 30 minutes under nitrogen atmosphere. The mixture aforementioned is next sequentially heated to 200°C for 1-hour and to 285°C for 30 minutes to complete the nucleation and growth of the superparamagnetic iron oxide nanoparticles. After cooling to room temperature, the superparamagnetic iron oxide nanoparticles are collected by centrifugation at 6,000 rpm for 10 minutes, and purified with ethanol for 3 times.

The oxidized dextran with the aldehyde group (hereafter "Dex") is prepared as follows. The dextran (the molecular mass ranges from 5 kDa to 270 kDa) is dissolved in an aqueous oxidation buffer (0.5-10 mg ml-1, pH= 5.5) containing sodium periodate solution (10 mM) for 30 minutes at room temperature in dark for oxidation. To remove the sodium meta-periodate, the Dex is dialyzed using Amicon (Mw=3 kDa), re-dispersed, and lyophilized using Lyophilizer (FreeZone 1L Benchtop Freeze Dry Systems, Labconco, Kansas). The structure of the Dex is characterized using a nuclear magnetic resonance (NMR), and the degree of modification of the Dex is characterized using a colorimetric aldehyde assay kit (MAK140, sigma).

In this experiment, the magnetic fucoidan nanoparticle is fabricated as follows. 0.5 mg/ml of the fucoidan (extracted from *Fucus vesiculosus*) and 0.5 mg/ml of the Dex are mixed as the hydrophilic phase solution. 2 mg of the IO is dissolved in 0.2 ml of the dichloromethane as the organic phase solution. The hydrophilic phase solution and the organic phase solution are mixed and then emulsified with 120 W for 50 seconds using a homogenizer (Double Eagle Enterprise Co, Ltd) to obtain an emulsion. After the dichloromethane is removed using rotary evaporator, the magnetic fucoidan nanoparticles (hereafter "IO@FuDex") are purified by using the magnetic selection equipment (MagniSort, eBioscience). After depleting the excess materials, the IO@FuDex are re-suspended with DDW or 0.1 M PBS (pH= 6) for further surface modification. In addition, magnetic nanoparticles IO@Fu are fabricated using the same preparation method except the hydrophilic phase solution is 0.5 mg/ml of the fucoidan. Magnetic nanoparticles IO@Dex are also fabricated using the same preparation method except the hydrophilic phase solution is 0.5 mg/ml of the Dex. The structures of the IO@FuDex, the IO@Fu and the IO@Dex are analyzed by the SEM and the TEM. The ZP and the particle size of the IO@FuDex, the IO@Fu and the IO@Dex are analyzed by the particle analyzer. The magnetic analysis of the IO@FuDex is analyzed by a superconducting quantum interference magnetometer.

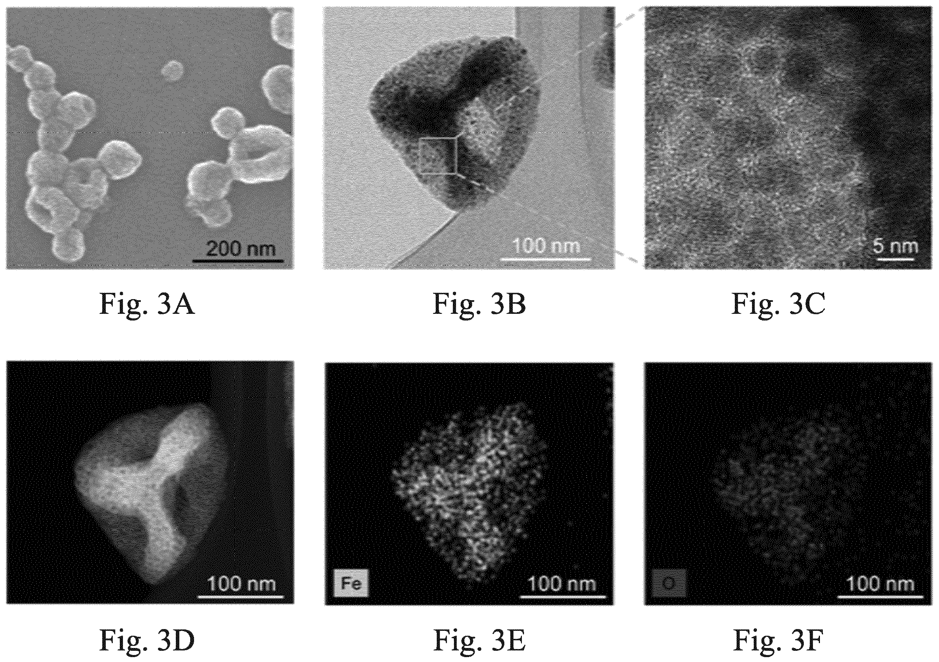

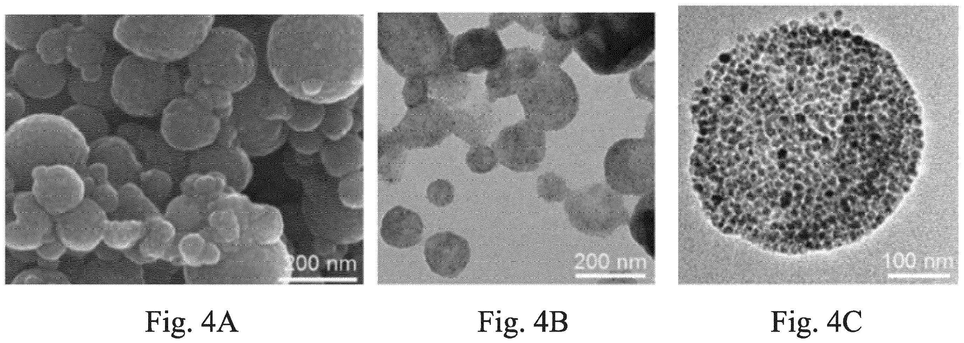

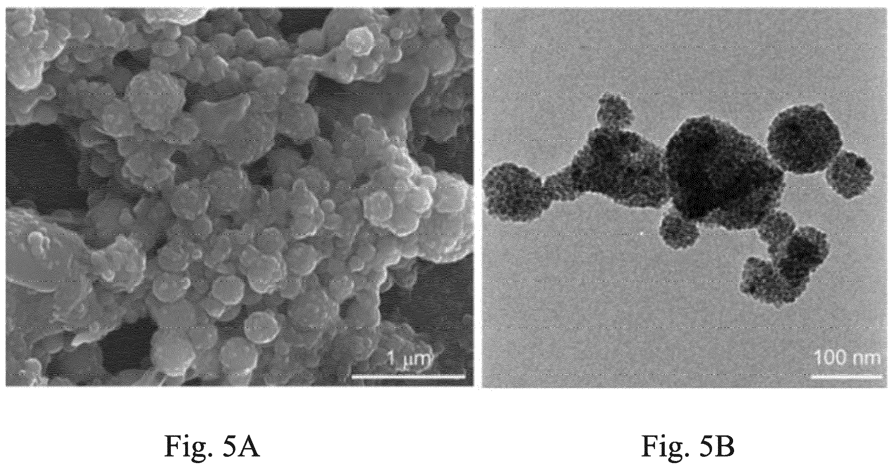

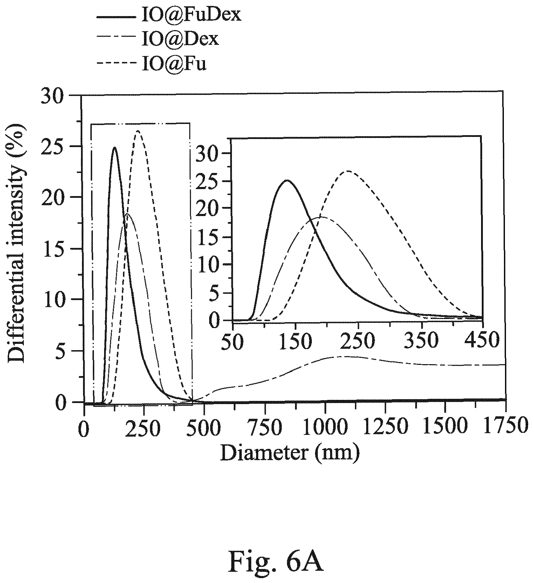

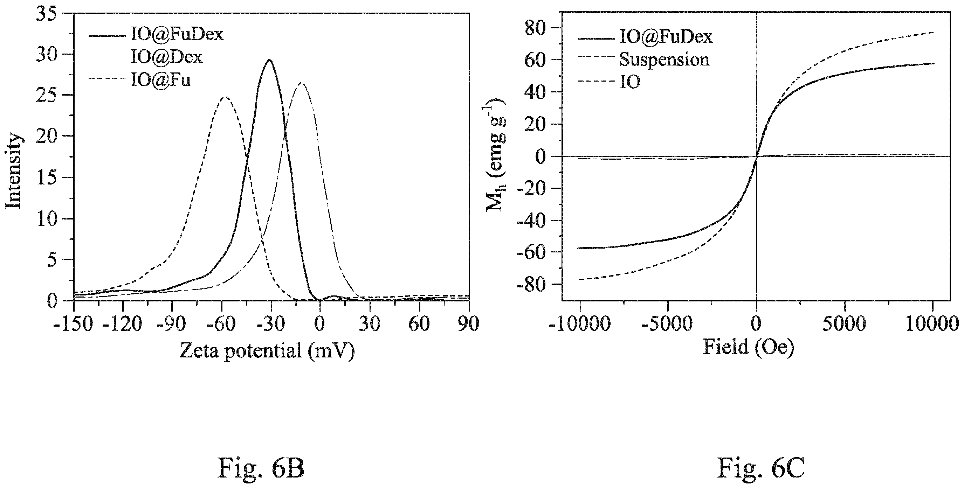

Please refer to Figs. 3A to 6C. Figs. 3A to 3F show structural analysis results of the IO@FuDex, wherein the Fig. 3A is a scanning electron micrograph of the IO@FuDex, and Figs. 3B to 3F are transmission electron micrographs of the IO@FuDex. Figs. 4A to 4C show structural analysis results of the IO@Fu, wherein Figs. 4A and 4B are scanning electron micrographs of the IO@Fu, and Fig. 4C is a transmission electron micrograph of the IO@Fu. Figs. 5A and 5B show structural analysis results of the IO@Dex, wherein Fig. 5A is a scanning electron micrograph of the IO@Dex, and Fig. 5B is a transmission electron micrograph of the IO@Dex. Figs. 6A to 6C show stability analysis results of the IO@FuDex, wherein Fig. 6A shows hydrodynamic size distribution of the IO@FuDex, the IO@Fu and the IO@Dex, Fig. 6B shows ZP analysis result of the IO@FuDex, the IO@Fu and the IO@Dex, and Fig. 6C shows magnetization analysis result of the IO@FuDex and IO.

In Fig. 3A, the IO@FuDex is the sphere and collapsed under high-vacuumed condition under SEM analysis, which represents that the IO@FuDex is a hollow structure. In Fig. 3B, the dark contrast caused by the overlaying shells can be observed under TEM analysis. Fig. 3C is a transmission electron micrograph at a partial enlarged view of the shell. In Fig. 3C, many superparamagnetic iron oxide nanoparticles with the size of about 5 nm can also be observed, which proves that the superparamagnetic iron oxide nanoparticle is the structure of the shell. Under the dark field observation in Fig. 3D and the elemental distribution detection in Figs. 3E and 3F, the hollow structure and the distribution of iron oxide in the IO@FuDex can be obvious observed again.

In Figs. 4A to 5B, the IO@Fu exhibits homogeneous structure as the IO@FuDex, while the IO@Dex is not stable. The IO@Dex tends to aggregate during the evaporation process, resulting in a wide distribution of particle size and random morphological appearance.

In Figs. 6A and 6B, the IO@FuDex is monodispersed in the fluid with the particle size ranging from 80 nm to 350 nm. An average particle size of the IO@FuDex is 141.5 nm, which is smaller than the average particle size of IO@Fu (241 nm). Both the IO@Fu and the IO@FuDex, mainly stabilized by the fucoidan, exhibit strong negative ZP due to the sulfate in the fucoidan, with IO@FuDex co-stabilized by the Dex having lower ZP (-32.8 mV) than the IO@Fu (-58.4 mV). Therefore, the strong repellent force between the IO@FuDexs can keep the colloid stable and well-dispersed. In Fig. 6C, the IO has a very high saturation magnetization per unit weight. The saturation magnetization value of the IO@FuDex is slightly lower due to its inclusion of the IO, the fucoidan and the Dex, but the IO@FuDex still possesses strong magnetic properties with 57.5 emu g$^{-1}$ of magnetization. The result indicates that strength of the magnetization of the IO@FuDex does not decrease due to the formation of a composite structure, which facilitates the magnetic purification using a magnetic separation equipment for obtaining high yields of the IO@FuDex.

The IO@FuDex is further fabricated by the Dex with different molecular mass. The IO@FuDex is fabricated by the aforementioned preparation method, but the Dex in the hydrophilic phase solution provided in Step 310 of Fig. 2 is prepared from the dextran with the molecular mass ranging from 5 kDa to 270 kDa, respectively. Then the Dex and the fucoidan are mixed in the weight ratio of 1: 0.2. Details of the remaining Steps 320 to 340 are substantially the same as described in Experiment 1.1, and the details are not described herein again. Then the particle size of the IO@FuDex are analyzed by the particle analyzer, the results are shown in the following Table 1:

Table 1. The average particle size of the IO@FuDex fabricated by different molecular mass of the Dex

| Molecular mass of dextran (kDa) | average particle size (nm) |
| --- | --- |
| 5 | 132 ± 14.5 |
| 12 | 130 ± 9.8 |
| 25 | 141 ± 16.2 |
| 50 | 162 ± 23.6 |
| 80 | 176 ± 26.8 |
| 150 | 203 ± 48.6 |
| 270 | 264 ± 32.6 |

The IO@FuDex is further fabricated by different weight ratio of the fucoidan and the Dex. The IO@FuDex is fabricated by the aforementioned preparation method, but the fucoidan and the Dex in the hydrophilic phase solution provided in Step 310 of Fig. 2 is mixed in the weight ratio of 1: 0.1 to 1: 4, respectively. Details of the remaining Steps 320 to 340 are substantially the same as described in Experiment 1.1, and the details are not described herein again. Then the particle size of the IO@FuDex are analyzed by the particle analyzer, the results are shown in the following Table 2:

Table 2. The average particle size of the IO@FuDex fabricated by different weight ratio of the fucoidan and the Dex

| the fucoidan : the Dex (weight ratio) | average particle size (nm) |
|---|---|
| 1 : 0.1 | 145 ± 6.9 |
| 1 : 0.2 | 153 ± 11.6 |
| 1 : 1 | 130 ± 9.8 |
| 1 : 2 | 141 ± 16.2 |
| 1 : 3 | 162 ± 23.6 |
| 1 : 4 | 176 ± 26.8 |

To test whether the IO@FuDex lyophilized and then re-dispersed in an aqueous solution remains the same structure, the fabricated IO@FuDex is further lyophilized by the lyophilizer to form a powdery crystal in this experiment. The lyophilized powdery crystal is re-dispersed in an aqueous solution, and the structure of the IO@FuDex before and after lyophilized are observed under the TEM analysis.

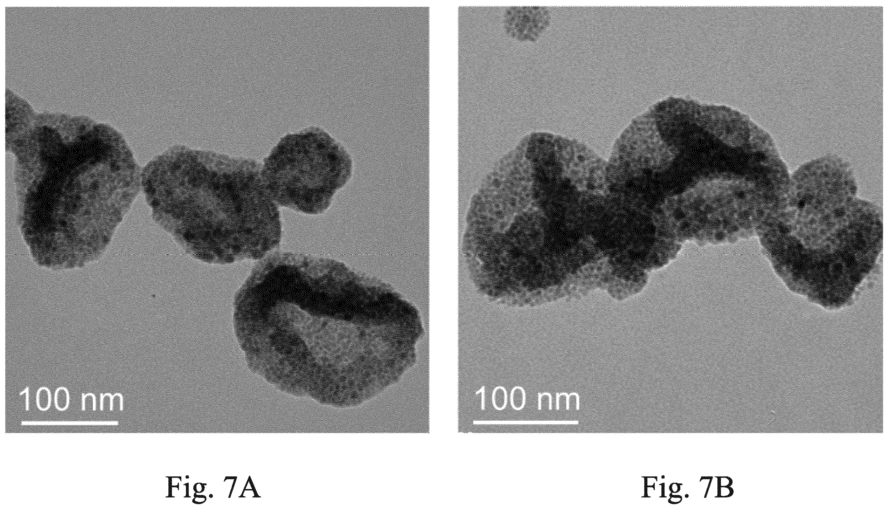

Please refer to Figs. 7A and 7B. Fig. 7A is a transmission electron micrograph of the IO@FuDex before lyophilized. Fig. 7B is a transmission electron micrograph of the IO@FuDex after lyophilized. The result shows that the structure of the IO@FuDex before lyophilized is a hollow sphere with the particle size ranging from 80 nm to 350 nm. The lyophilized powdery crystal can be rapidly re-dispersed in the aqueous solution. The structure of IO@FuDex re-dispersed remains as the hollow sphere with the particle size consistent with the IO@FuDex before lyophilized under the TEM analysis. These results indicate that the stability of the IO@FuDex is excellent.

### 1.2 Synthesis of examples 1-3

The immunomagnetic composition of the present disclosure is further fabricated under the optimal condition described above for fabricating the IO@FuDex. The fabricated IO@FuDexs are incubated with different antibodies in the buffer (0.1 M, pH= 6) containing 5 μM sodium cyanoborohydride performing antibody immobilization for 4-6 hours at 4°C. A dynamic process of the immobilization starts as the aldehydes in the Dex form Schiff bases with the primary amines on antibodies, and the immobilization further becomes chemically stabilized after undergoing reductive amination reaction with the use of sodium cyanoborohydride. The fabricated immunomagnetic compositions are purified using the magnetic separation equipment. Please refer to following Table 3, which represents the antibodies used in examples 1-3. The antibodies used in the example 1 are the CD3 antibody and the CD28 antibody, the antibody used in the example 2 is the PD-L1 antibody, and the antibodies used in the example 3 are the PD-L1 antibody, the CD3 antibody and the CD28 antibody.

Table 3

|  | PD-L1 antibody | CD3 antibody | CD28 antibody |
|---|---|---|---|
| IO@FuDex | - | - | - |
| example 1 | - | + | + |
| example 2 | + | - | - |
| example 3 | + | + | + |

The fabricated examples are analyzed for the structure and elemental analysis by the TEM and for elemental composition in the example 3 by X-ray photoelectron spectroscopy (XPS).

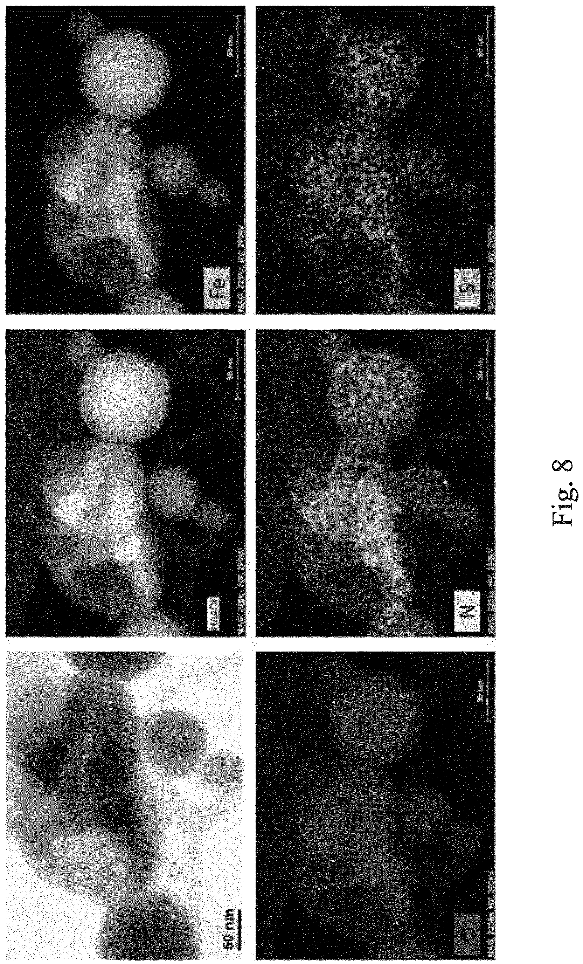

Please refer to Fig. 8, which shows structural analysis results of the example 3. In Fig. 8, the structure of the example 3 is a spherical hollow structure with the particle size of about 80 nm to 350 nm. The elemental analysis of the electron microscopy revealed that the outer layer of the example 3 shows a uniform nitrogen signal (N) because of the presence of the antibody. Before immobilizing the antibody, the composition of the IO@FuDex is only sugars and iron oxide, and there is no material with nitrogen. After immobilizing the antibody, the antibody has many amino and peptide bonds with a large amount of nitrogen. The result indicates that the immunomagnetic composition of the present disclosure includes the antibody.

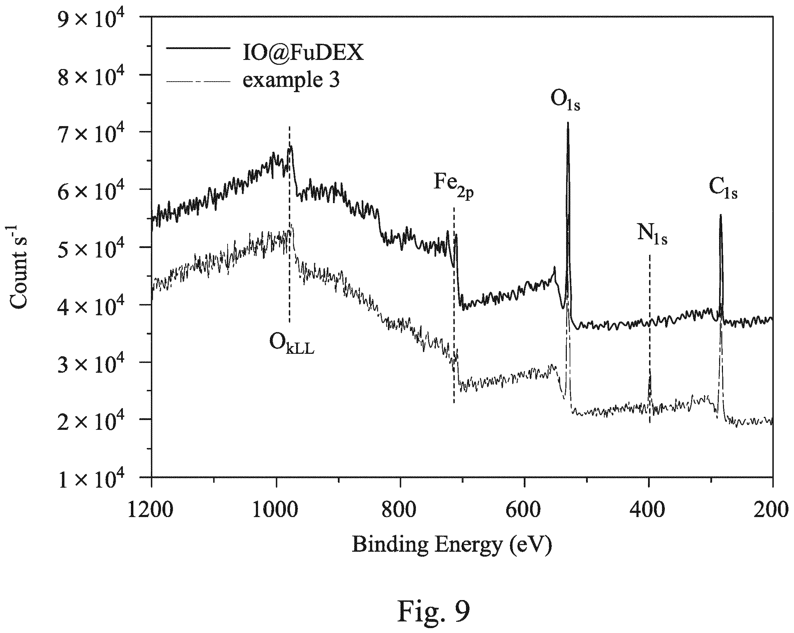

Please refer to Fig. 9, which shows XPS analysis results of the example 3. Compared with the IO@FuDex, the example 3 has an added signal with a bonding energy at 399 eV, which is a nitrogen signal. The result indicates that the antibody is successfully immobilized onto the example 3.

**1.3 Synthesis and structure confirmation of example 5**

The immunomagnetic composition is shown as the hollow shape, hence the active substance, such as the cytokine or the anti-cancer drug can be encapsulated in the core of the immunomagnetic composition. The active substance used in this experiment is Interleukin-2 (IL-2) for preparing the example 5 including the IL-2.

In this experiment, the example 5 is fabricated as follows. 0.5 mg/ml of the fucoidan, 0.5 mg/ml of the Dex and 50 $\mu$g are mixed as the hydrophilic phase solution. 2 mg of the IO is dissolved in 0.2 ml of the dichloromethane as the organic phase solution. The hydrophilic phase solution and the organic phase solution are mixed and then emulsified with 120 W for 50 seconds using a homogenizer to obtain the emulsion. After the dichloromethane is removed using rotary evaporator, the example 5 is purified by using the magnetic selection equipment. The structure of the fabricated example 5 is analyzed by the SEM. The particle size of the fabricated example 5 is analyzed by the particle analyzer.

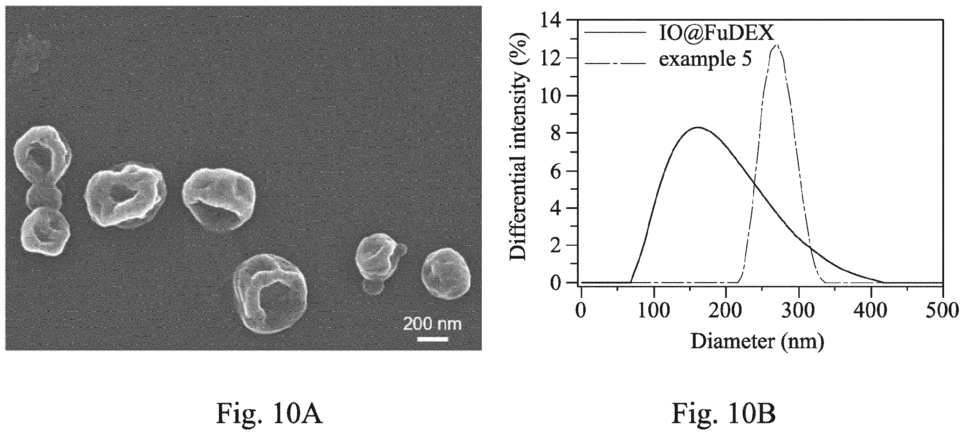

Please refer to Figs. 10A and 10B, which are structural analysis results of example 5. Fig. 10A is a scanning electron micrograph of the example 5, and Fig. 10B shows hydrodynamic size distribution of the example 5. The entrapment efficiency of the IL-2 in the example 5 is 99.93%. Because the IL-2 fills the core, compared with the IO@FuDex with the average particle size of 161.2 nm, the average particle size of the example 5 is increased to 356.2 nm. The result indicates that the active substance can be further encapsulated in the core of the immunomagnetic composition of the present disclosure. Other active substances, such as Doxorubicin, Docetaxel, Paclitaxel or Astaxanthin (ASTX), also can be encapsulated in the immunomagnetic composition of the present disclosure using the same preparation method.

**1.4 Targeting ability analysis of the immunomagnetic composition of the present disclosure**

In this experiment, the fabricated examples 1-3 are performed the targeting ability analysis and the cell association ability analysis to examine whether the immunomagnetic composition of the present disclosure can reverse the decline of T-cell immunity and enhance tumor regression in mice. The aggressive and triple negative breast cancer cell line (4T1) with metastatic capacity and PD-L1 expression is selected as the experimental model. To achieve the observation of examples 1-3 under fluorescence microscopy analysis, quantum-dot (QD) is further incorporated into the structure of the examples 1-3 of fluorescence microscopy analysis group, respectively.

To assess targeting behavior *in vitro,* the examples 1-3 are incubated with the 4T1-Luc cells ($4 \times 10^5$) in the presence of bovine serum albumin (BSA) under 4°C for 30 minutes, and analyzed using flow cytometry (Novocyte Flow Cytometer, ACEA Biosciences). To access the cell association behavior, IO@FuDex incorporated with the QD and the example 2 incorporated with the QD are incubated with the 4T1-Luc cells for 1, 4, 12 and 24 hours, and either analyzed using flow cytometry or fluorescent microscopy (Carl Zeiss, Thornwood).

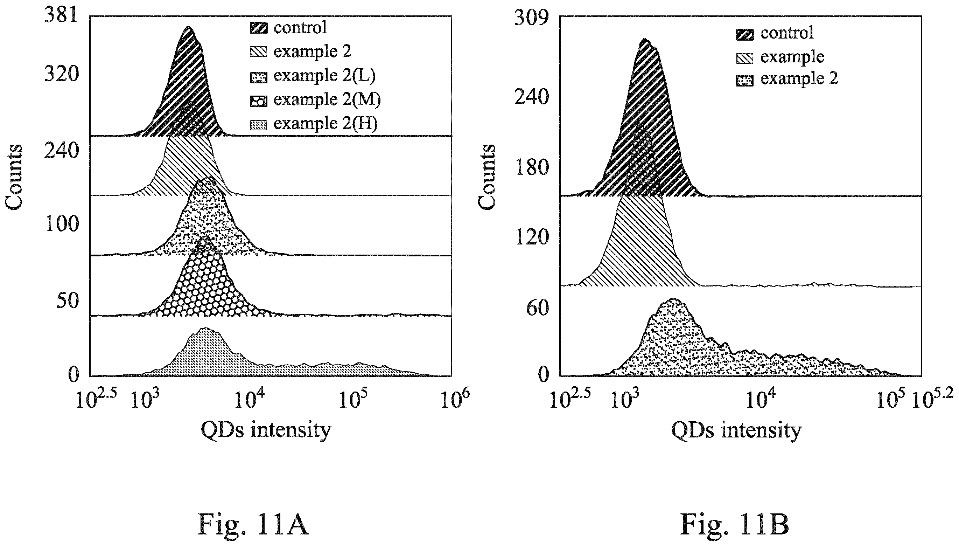

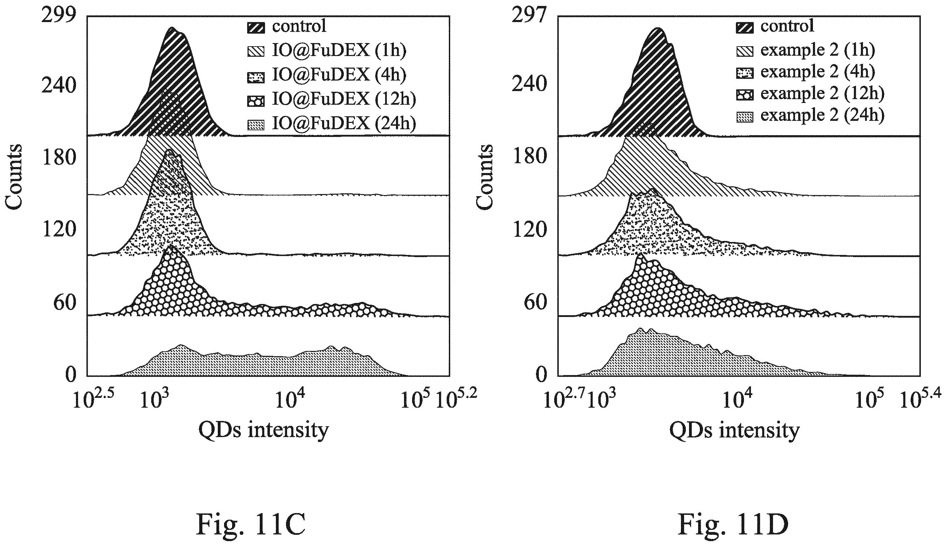

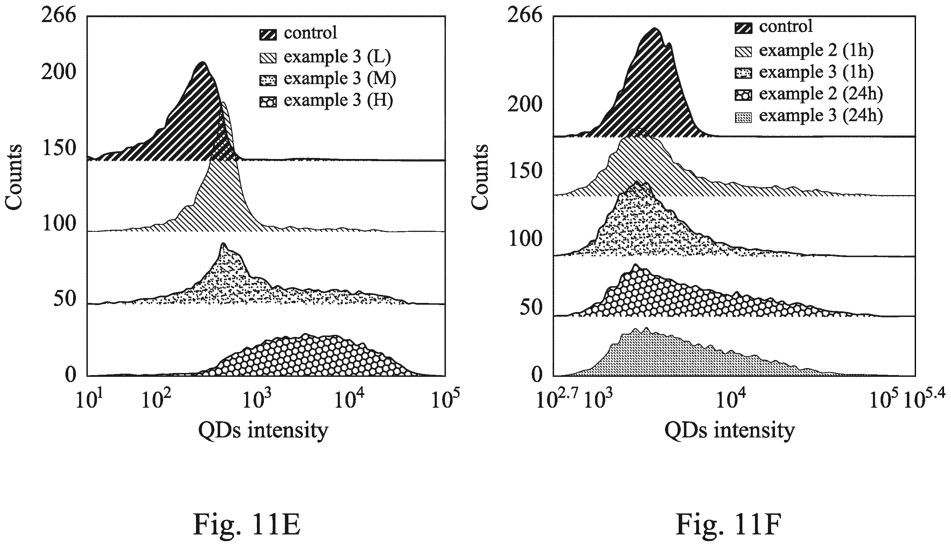

Please refer to Figs. 11A to 11F, which show targeting ability and cell association ability analysis results of the immunomagnetic composition of the present disclosure. Fig. 11A shows the targeting ability analysis results of the example 2 with the PD-L1 antibody conjugation concentration of 1.4 $\mu$g/ml (low concentration, L), 7 $\mu$g/ml (medium concentration, M) and 35$\mu$g/ml (high concentration, H), respectively. Fig. 11B shows the targeting ability analysis results of the example 2 and an IgG-labeled IO@FuDex. Fig. 11C shows the 4T1 cell association ability analysis results of the IO@FuDex under 1, 4, 12 and 24 hours incubation. Fig. 11D shows the 4T1 cell association ability analysis results of the example 2 under 1, 4, 12 and 24 hours incubation. Fig. 11E shows the targeting ability analysis results of the example 3 with the CD3 antibody/CD28 antibody conjugation concentration of 1.4 $\mu$g/ml (low concentration, L), 7 $\mu$g/ml (medium concentration, M) and 35$\mu$g/ml (high concentration, H), respectively. Fig. 11F shows the 4T1 cell association ability analysis results of the example 2 and example 3 under 1 hour and 24 hours incubation.

In Fig. 11A, after 1-hour incubation, 4T1 cells associate with the example 2 (H) exhibits the highest median fluorescence index (MDI). It indicates that the amount of the example 2 labeled onto PD-L1-expressed 4T1 cells is strongly correlated with the surface antibody density. Thus, the PD-L1 antibody conjugation concentration in the subsequent example 2 is high concentration (35 $\mu$g/ml). In Fig. 11B, no MDI shift is observed for the IgG-labeled IO@FuDex, demonstrating the affinity to 4T1 cells is derived from the PD-L1 antibody instead of any non-specific interaction of IO@FuDex.

In Figs. 11C and 11D, the amount of cell association is time-dependent for the IO@FuDex, which shows obvious cell uptake after 12 hours incubation. In contrast, the example 2 achieves cell association within 1-hour incubation, follows by a slow shift in MDI, which indicates more cell association of the example 2 as the incubation time increased. The result indicates that the immobilization of the PD-L1 antibody changes 4T1 cell association ability of the IO@FuDex.

Subsequently, to achieve the goal that the present disclosure can simultaneously be the immune checkpoint inhibitor and the T cell expansion agent, the example 3 is the immunomagnetic composition immobilizing the CD3 antibody, CD28 antibody and the PD-L1 antibody. Fig. 11E shows the targeting ability analysis results of the example 3 with different CD3 antibody/CD28 antibody conjugation concentration. In Fig. 11E, after 1-hour incubation, 4T1 cells associate with the example 3 (H) exhibits the highest MDI, indicating strong affinity of the example 3 (H) to CD8+ T cell. In Fig. 11F, the example 3 has similar 4T1 cell association behavior to the example 2, indicating that the immobilization of multiple antibodies does not impair the affinity of the immunomagnetic composition to 4T1 cells.

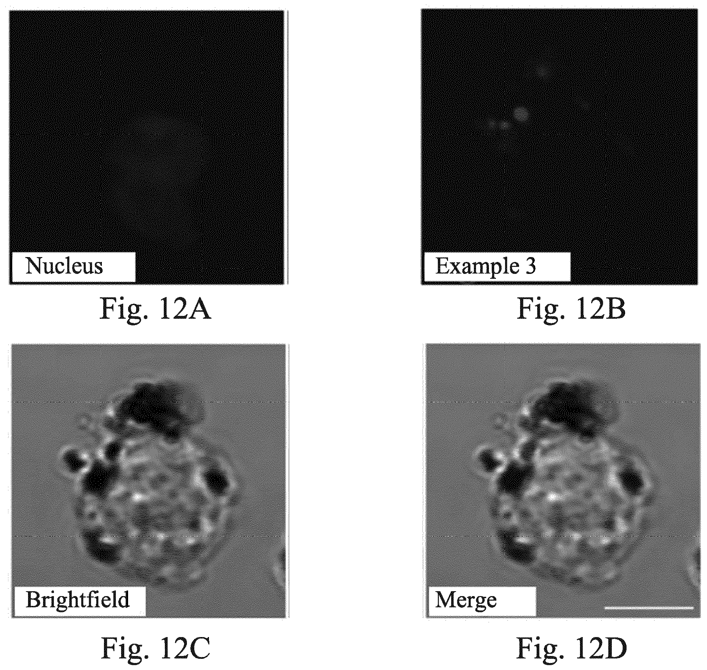

Please refer to Figs. 12A to 12D, which show cell association ability analysis results of the example 3 binding to CD8+ T cell. In this experiment, the CD8+ T cells are incubated with the example 3 incorporated with the QD for 30 minutes, and then the CD8+ T cells are fixed, stained using Alexa Fluor 488 Phalloidin and DAPI, and observed using confocal for confirming the position of the example related to the CD8+ T cells. Fig. 12A represents the position of the nucleus, Fig. 12B represents the position of the example 3, Fig. 12C is a micrograph in the light field, and Fig. 12D is a merged micrograph, where the scale represents the length of 5 $\mu$m. In Figs. 12A to 12D, a plurality of the example 3 can be observed in the CD8+ T cells, indicating that the example 3 can bind to the CD8+ T cells.

## II. Use of the immunomagnetic composition

### 2.1 Therapeutic effect of the immunomagnetic composition of the present disclosure on treatment of cancer

The experiments in this part further demonstrate whether the immunomagnetic composition of the present disclosure has the therapeutic effect on cancer and whether it can be accumulated in the tumor via magnetic navigation (MN) in the breast cancer metastasis mouse model and the colorectal cancer mouse model.

### 2.1.1 Therapeutic effect of the immunomagnetic composition of the present disclosure on treatment of lung metastasis of breast cancer

The 4T1-Luc tumor-bearing mice with a luciferase gene that stably express a biological luminescent enzyme (provided by the Center for Molecular Medicine, University Hospital of China Medical University) are used as the animal model in this experiment. The immunomagnetic composition including the 125I-labled examples 1-3 is administered via the right femoral vein to the 4T1-Luc tumor-bearing mice, respectively. In addition, the example 4, which includes the 125I-labled example 3 and a superficial round-shape magnet (diameter=0.5 cm, 0.5 Tesla) for the magnetic navigation, is included in this experiment as the kit for treating cancer. The dynamic intratumoral accumulation of the example 3 and the example 4 are monitored using single-photon emission computed tomography (SPECT).

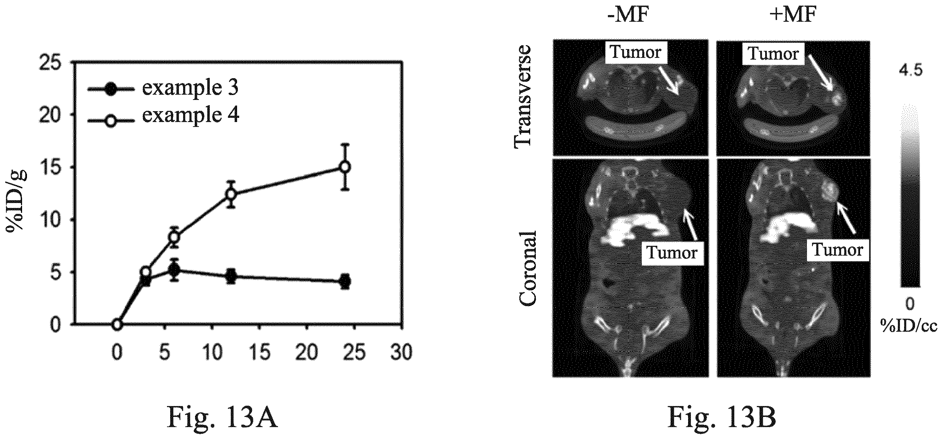

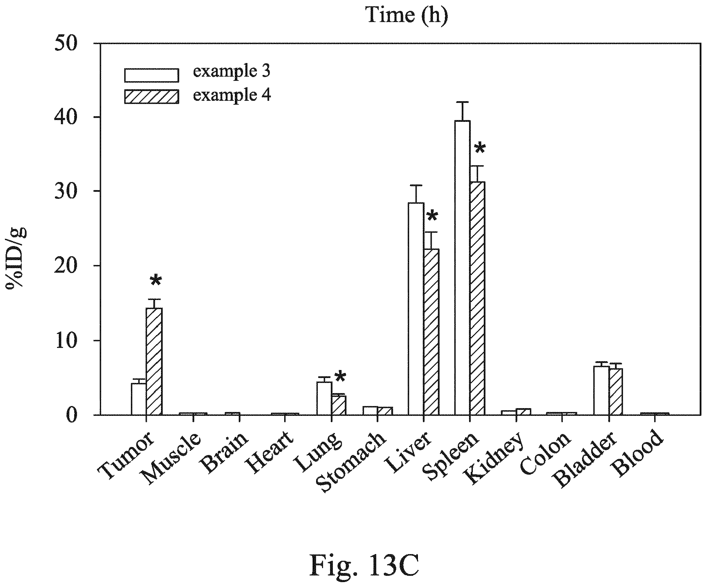

Please refer to Figs. 13A to 13C, which show analysis results of nanomedicine accumulation in tumor of the kit for treating cancer of the present disclosure. Fig. 13A shows time-activity curves of the 125I-labled example 3 or the example 4 in the 4T1-Luc tumor-bearing mice at the time points of 0, 4, 6, 12, and 24 hours, wherein n = 4. Fig. 13B shows whole-body single photon emission computed tomography images of the 4T1-Luc tumor-bearing mice at 24 hours after administering the 125I-labled example 3 or the example 4. Fig. 13C shows biodistribution quantitative profiles of the 4T1-Luc tumor-bearing mice at 24 hours after intravenous infusion of the 125I-labled example 3 or the example 4, wherein n = 4, and marked asterisks * represents statistically significant difference ($p < 0.05$).

In Fig. 13A, the tumor accumulation of the example reached a maximum concentration of (5.08 % injected dose per gram tissue ID/g) at 6 hours postadministration. In contrast, a continuous accumulation of the example 4 within 24 hours is revealed by facilitating the dose localization at the tumor via the magnetic navigation, resulting in a 3.6-fold higher % ID/g than the example 3 at 24 hours postadministration. In Fig. 13B, a different biodistribution pattern of the radioisotope between the example 3 and the example 4 is noticed at 24 hours postadministration of the example 3 and the example 4. In Fig. 13C, biodistribution quantitative profiles demonstrate that the example 3 and the example 4 are mostly accumulated at tumor, liver, spleen, and bladder, with minor accumulation at muscle, brain, heart, lung, stomach, kidney, colon, and blood (less than 5 % ID/g). More importantly, compared with the example 3, the magnetic navigation in the example 4 not only increases the tumor accumulation, but significantly reduces the systemic accumulation in the liver and the spleen, indicating that the example 4 can be effectively centralized to the site of action.

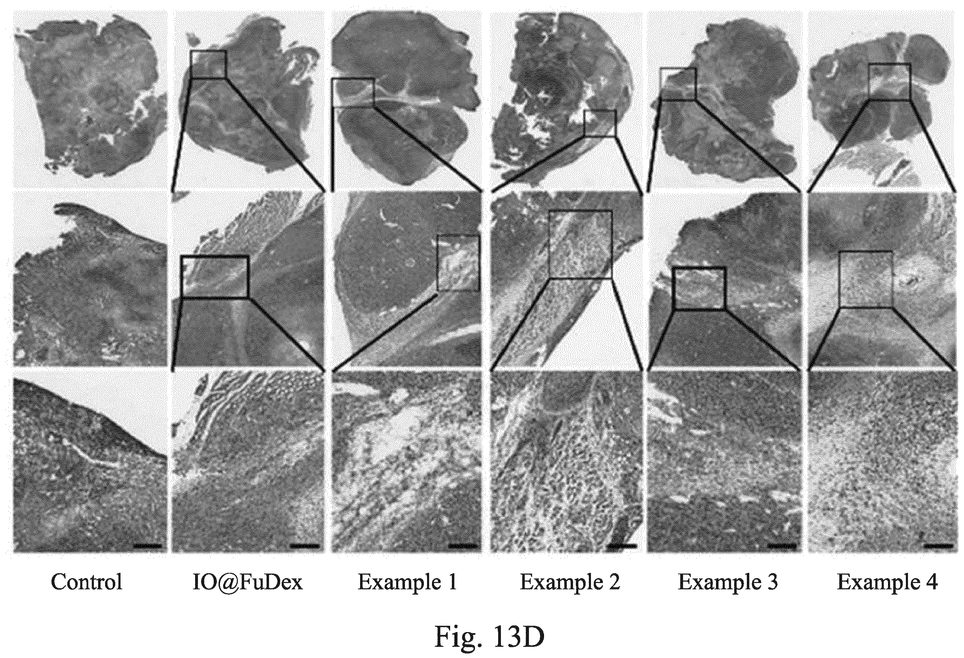

In this experiment, the 4T1-Luc tumor-bearing mice are further administrated the IgG (control), the IO@FuDex and the examples 1-4 via the right femoral vein. The administrations are started on day 8 after tumor implantation. The administration method is three times at intervals of 4 days (q4dx3). Fig. 13D shows hematoxylin and eosin stain with Prussian blue stain photographs of the 4T1-Luc tumor-bearing mice at 24 hours postadministration of the examples 1-4, wherein the scale represents the length of 50 $\mu$m. In Fig. 13D, dispersed Prussian blue stains can be observed in the tumor tissue with the example 4 administration compared to other examples.

To confirm the therapeutic effect of the immunomagnetic composition and the kit for treating cancer of the present disclosure have the therapeutic effect on the 4T1-Luc tumor-bearing mice, the 4T1-Luc tumor-bearing mice are further administrated the IgG (control), the IO@FuDex and the examples 1-4 via the right femoral vein. The administration was started on day 8 after tumor implantation. The administration method is three times at intervals of 4 days (q4dx3). The tumor volumes are monitored using a digital caliper (mitutoyo) every 2 to 3 days using the following Equation I:

$$\text{Tumor volume (mm}^3) = \frac{W^2 \times L}{2} \quad \cdots\cdots\cdots\cdots\cdots\cdots\cdots\text{Equation I;}$$

The bioluminescence assessment is analyzed by a non-invasion *in vivo* imaging system (IVIS 200 System, Xenogen). The survival rate of the 4T1-Luc tumor-bearing mice is analyzed using Kaplan-Meier survival analysis.

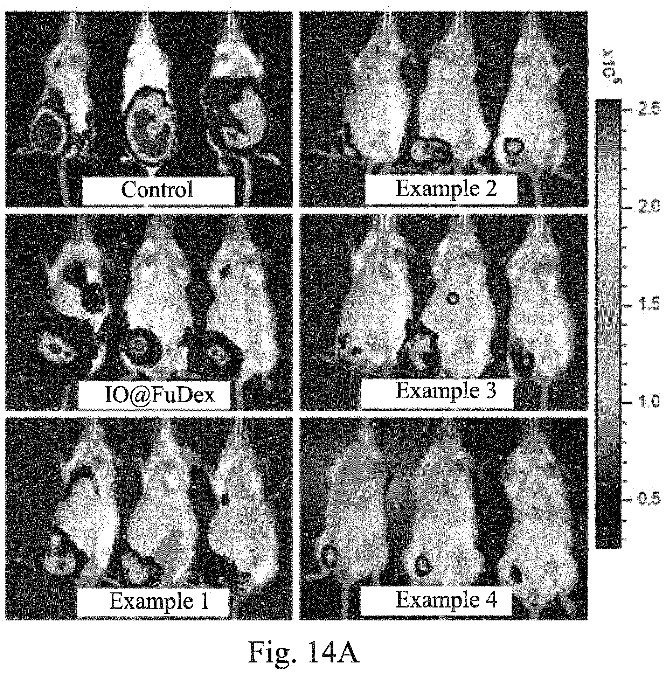

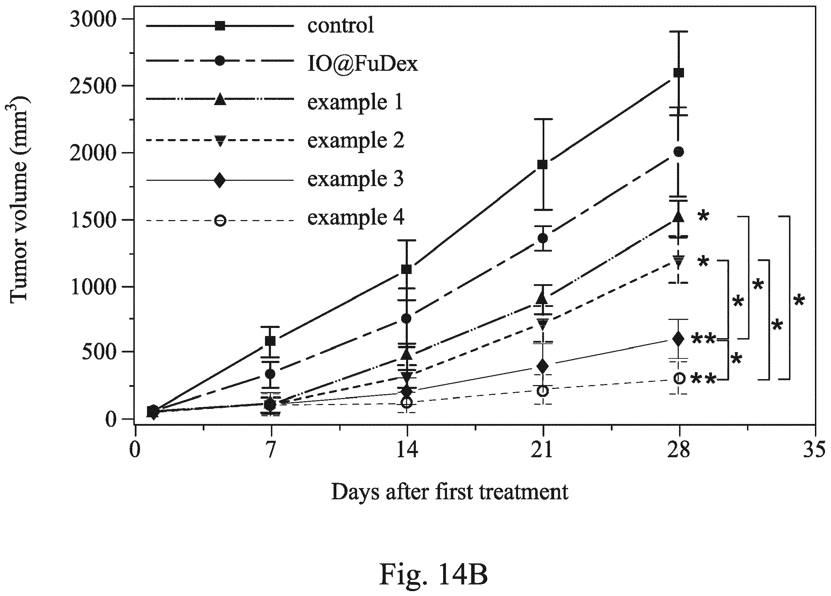

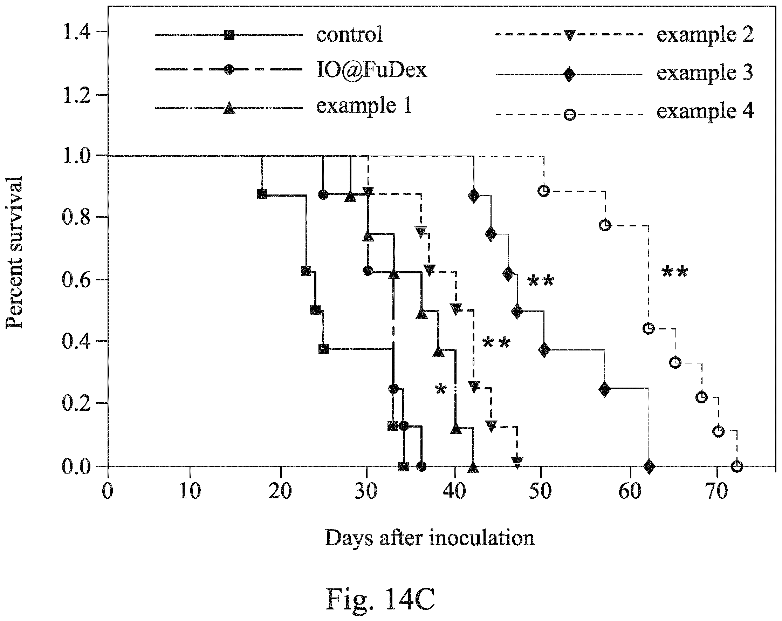

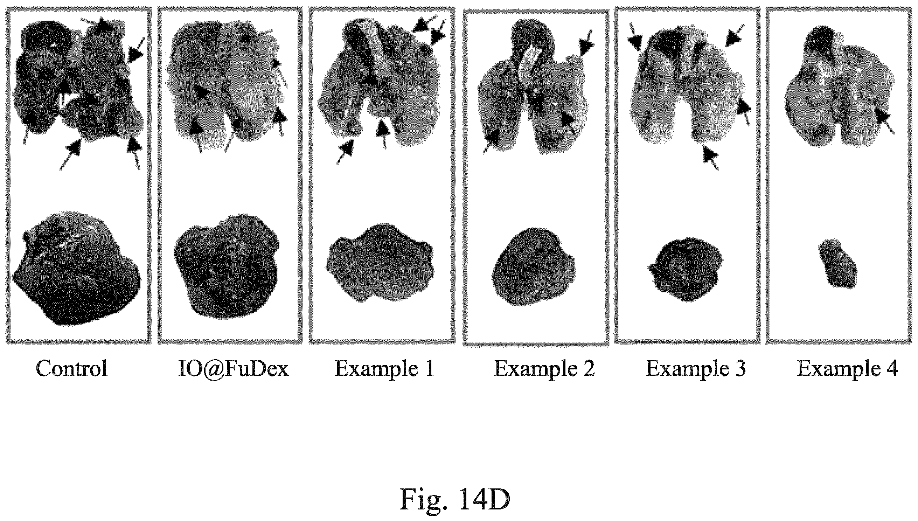

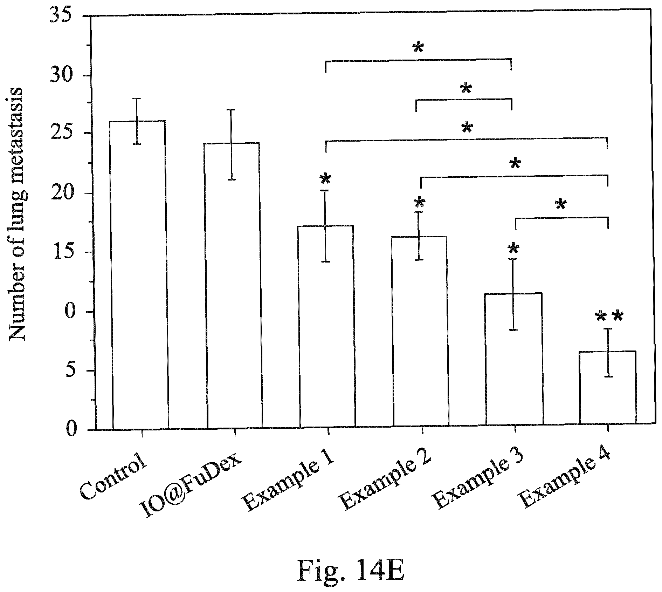

Please refer to Figs. 14A to 14E, which show analysis results of cancer cell proliferation inhibiting capacity and anti-metastasis capacity in the 4T1-Luc tumor-bearing mice of the immunomagnetic composition and the kit for treating cancer of the present disclosure. Fig. 14A shows the IVIS system scanning images of the 4T1-Luc tumor-bearing mice at 24 hours postadministration of the examples 1-4. Fig. 14B is a chart showing tumor volume of the 4T1-Luc tumor-bearing mice administrated the examples 1-4. Fig. 14C shows survival curves of the 4T1-Luc tumor-bearing mice administrated the examples 1-4. Fig. 14D shows pictures of dissected tumors and lungs of the 4T1-Luc tumor-bearing mice administrated the examples 1-4. Fig. 14E is a chart showing lung metastases of the 4T1-Luc tumor-bearing mice administrated the examples 1-4.

In Figs. 14A and 14B, the IO@FuDex exhibited therapeutic effect on reducing tumor volumes compared to control group, but there is no statistically significant difference. The examples 1-4 show stronger anti-tumor effects with statistically significant difference (* represents $p < 0.05$ and ** represents $p < 0.01$), where the example 4 nearly inhibited tumor growth in 30 days. In Fig. 14C, the median survival time of the 4T1-Luc tumor-bearing mice administrated with the IgG, the IO@FuDex, the example 1, the example 2, and the example 3 are 24 days, 34 days, 34 days, 43 days and 44 days, while the median survival time of the 4T1-Luc tumor-bearing mice administrated with the example 4 significantly extends to 63 days. Although the dose of the PD-L1 antibody in the example 4 is only one percent of the dose of pure antibody administered in general, the example 4 is able to exhibit more excellent tumor inhibiting ability and extend the half-life by more than 2 times. Furthermore, the results in Fig. 14D indicate that all the examples exhibit anti-metastasis ability. As shown in Fig. 14D, the example 3 and the example 4 significantly prevent the tumor metastasis in lungs compared with other groups. In Fig. 14E, on average, fewer than 5 nodules of lung metastasis are discovered in the example 4 administrated 4T1-Luc tumor-bearing mice compared to over 20 metastases in lungs of the control administrated 4T1-Luc tumor-bearing mice. Thus, the administration including the immunomagnetic composition and the kit for treating cancer of the present disclosure evidently provide both tumor inhibition and anti-metastasis effect. However, the IO@FuDex does not show significant decrease in metastasis compared to the control group. As a result, we hypothesize that the anti-metastasis effect of the immunomagnetic composition and the kit for treating cancer of the present disclosure is not only inhibited by fucoidan but largely affected by the dynamic reactions in the tumor microenvironment.

**2.1.2 Therapeutic effect of the immunomagnetic composition of the present disclosure on treatment of colorectal cancer**

The CT-26 cell lines with the luciferase gene that stably express the biological luminescent enzyme (provided by the Center for Molecular Medicine, University Hospital of China Medical University) are utilized for building the colorectal cancer mouse model in this experiment. The IgG (control), the IO@FuDex and the immunomagnetic composition including the example 3 is administered via the right femoral vein to the CT-26 tumor-bearing mice, respectively. The example 4, which includes the example 3 and the superficial round-shape magnet (diameter=0.5 cm, 0.5 Tesla) for the magnetic navigation, is included in this experiment as the kit for treating cancer. In addition, the IO@FuDex cooperated with the superficial round-shape magnet for the magnetic navigation is included in this experiment as a comparative example 1. The administrations are started on day 8 after tumor implantation. The administration method is three times at intervals of 4 days (q4dx3). The tumor volumes are monitored using a digital caliper every 2 to 3 days using the Equation I. The bioluminescence assessment is analyzed by the IVIS System. The survival rate of the CT-26 tumor-bearing mice is analyzed using Kaplan-Meier survival analysis.

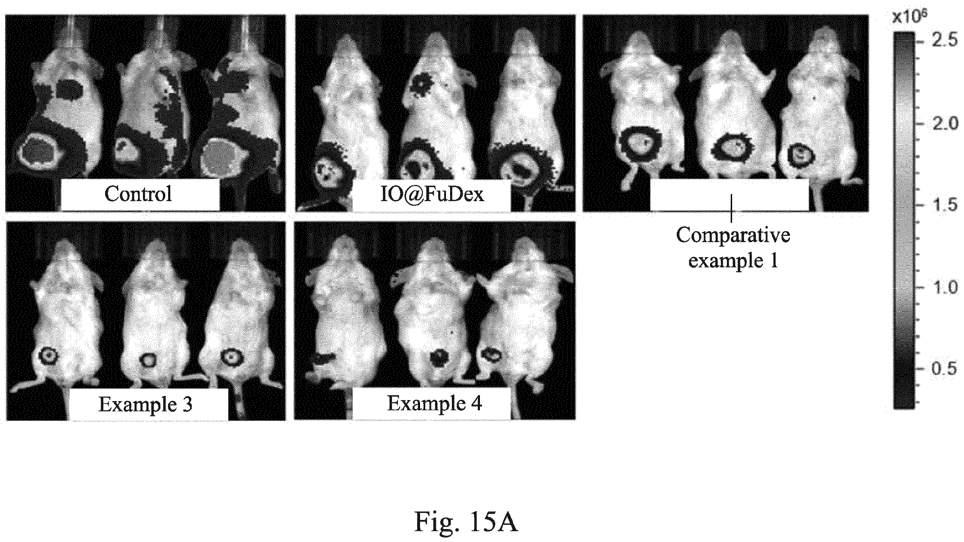

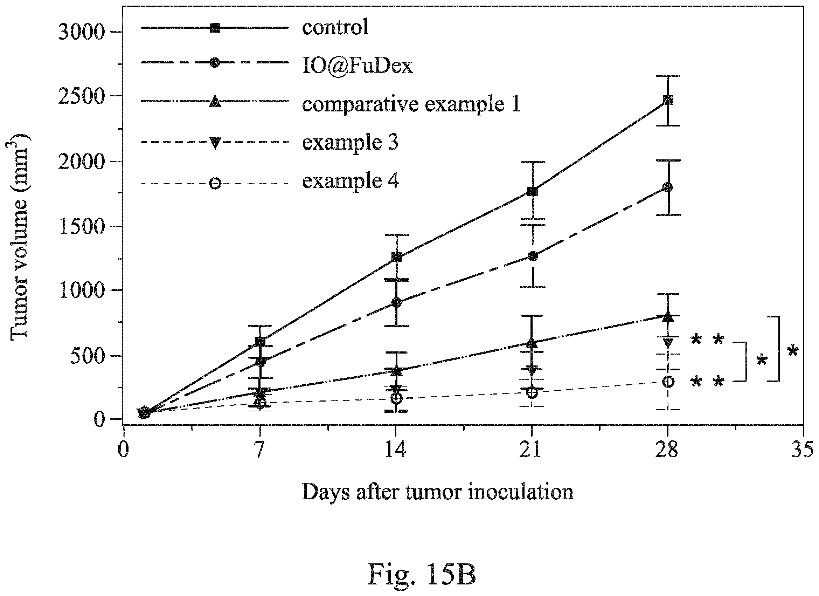

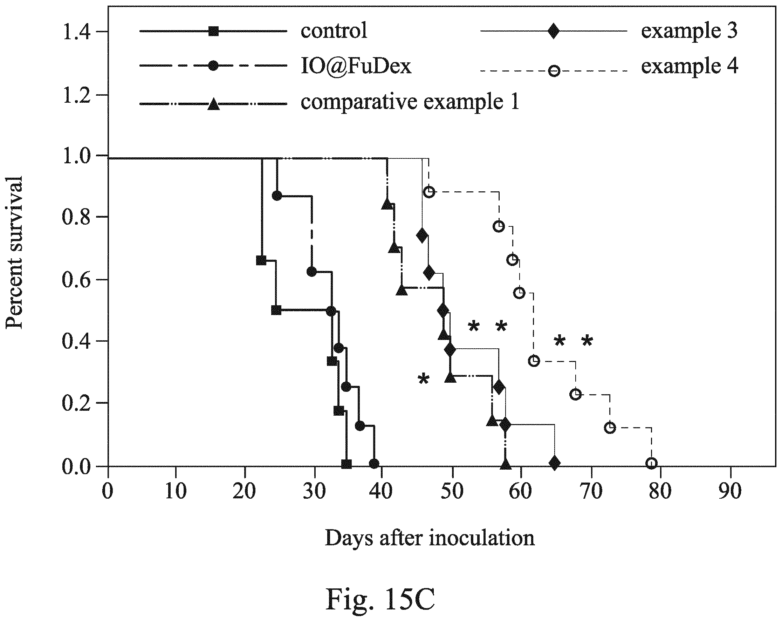

Please refer to Figs. 15A to 15C, which show analysis results of cancer cell proliferation inhibiting capacity in the colorectal cancer mouse model of the immunomagnetic composition and the kit for treating cancer of the present disclosure. Fig. 15A shows the IVIS system scanning images of the CT-26 tumor-bearing mice at 24 hours postadministration of the examples 3-4. Fig. 15B is a chart showing tumor volume of the CT-26 tumor-bearing mice administrated the examples 3-4. Fig. 15C shows survival curves of the CT-26 tumor-bearing mice administrated the examples 3-4.

In Figs. 15A and 15B, the IO@FuDex reduces the tumor volumes compared to control group, but there is no statistically significant difference. The comparative example 1, which administrates the IO@FuDex cooperated with the magnetic navigation, can increase the therapeutic effect of the IO@FuDex. The examples 3-4 show stronger anti-tumor effects with statistically significant difference (* represents $p < 0.05$ and ** represents $p < 0.01$), where the example 4 nearly inhibited tumor growth in 30 days. In Fig. 15C, the administration of the example 3 and the example 4 significantly prolongs the survival time of the CT-26 tumor-bearing mice. These results indicate the tumor inhibiting ability and the anti-metastasis effect of the pharmaceutical composition for treating cancer and the kit for treating cancer of the present disclosure again.

**2.2 Analysis of immune activation after administration of the immunomagnetic composition and the kit for treating cancer of the present disclosure**

The 4T1 tumor model is further used in this experiment to investigate the immune activation of the immunomagnetic composition and the kit for treating cancer of the present disclosure in the tumor microenvironment. The changes in the frequency of leukocytes in tumors, blood, ascites and spleens of the 4T1-Luc tumor-bearing mice from early (10 days) to late times of tumor growth (30 days) are monitored.

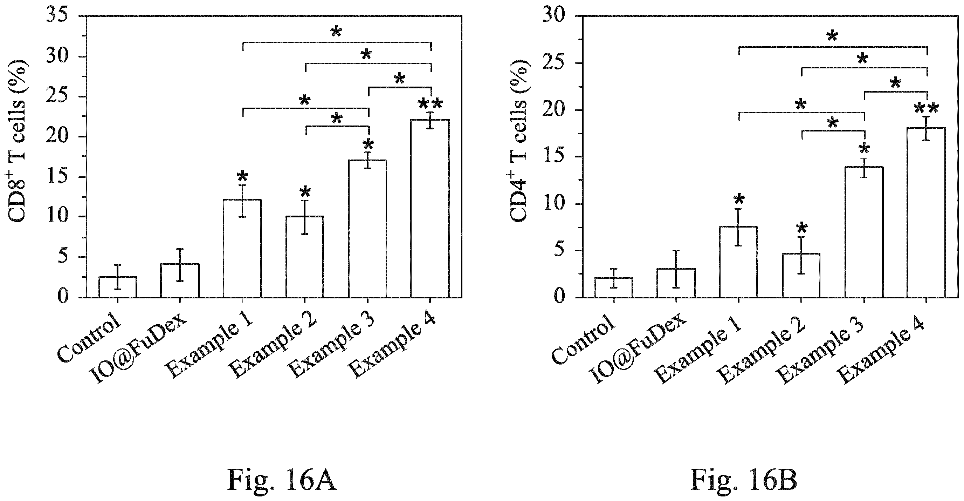

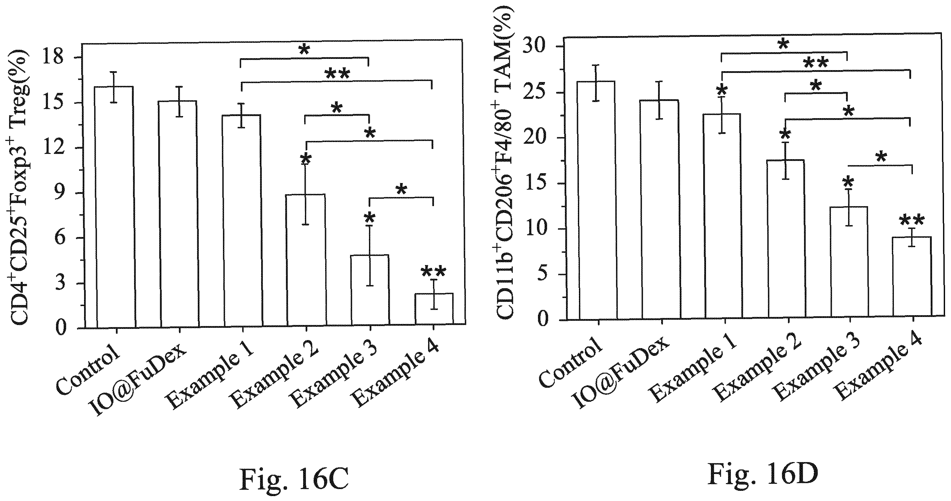

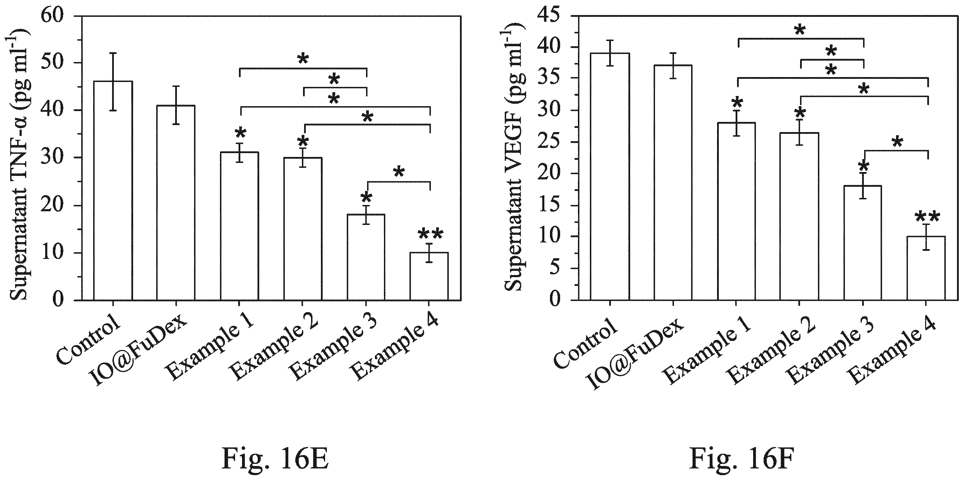

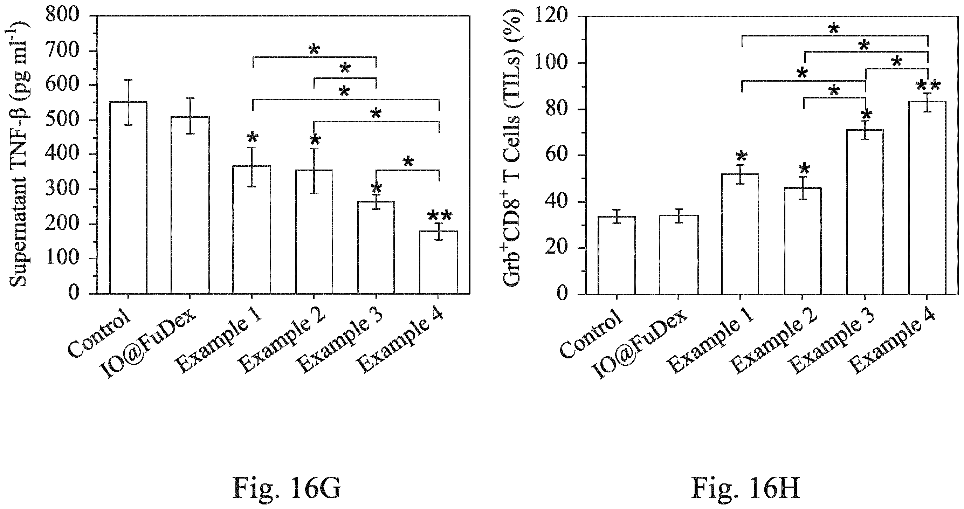

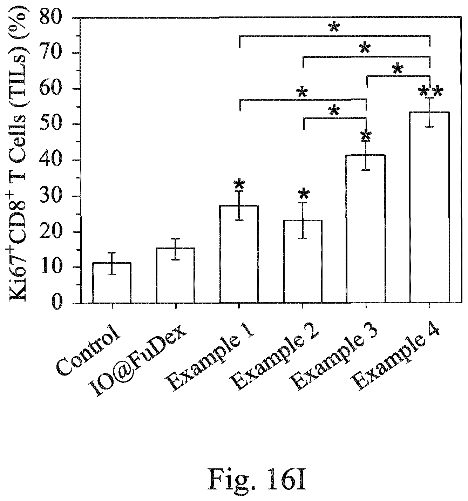

Please refer to Figs. 16A to 16I, which show analysis results of changes of tumor-infiltrating lymphocytes and cytokines in tumor microenvironment after administering the immunomagnetic composition and the kit for treating cancer of the present disclosure. In Figs. 16A and 16B, the number of antitumor lymphocytes, such as CD8$^+$ T cells and CD4$^+$ T cells, is significantly increased in the groups administrated the examples 1-4; in particularly in the group administered with the example 4 ($p < 0.01$). In Figs. 16C and 16D, the number of pro-tumor antitumor lymphocytes, such as CD4$^+$CD225$^+$Foxp3$^+$ Tregs (regulatory T cells) and CD11b$^+$CD206$^+$F4/80$^+$ TAM (tumor associated macrophages), is significantly reduced in the groups administrated the examples 1-4, especially the group administered with the example 4 can drastically reduce the number of regulatory T cells (Treg) ($p < 0.01$). Different groups of the 4T1-Luc tumor-bearing mice sera are collected to analyze the levels of TNF-$\alpha$, VEGF and TGF-$\beta$, secreted by the TAM. In Fig. 16E to 16G, the levels of the pro-inflammatory cytokines such as the TNF-$\alpha$, the VEGF and the TGF-$\beta$ can be significantly reduced in the groups administrated the examples 1-4; in particularly in the group administered with the example 4 ($p < 0.01$). In Figs. 16H and 16I, the degree of CD8$^+$T cells activation is assessed by the expression levels of intragranular granzyme B (GrB$^+$) and Ki67, which indicate that administration of the examples 1-4 can effectively activate CD8$^+$ TILs functions, especially the group administered with the example 4 ($p < 0.01$).

The aforementioned experiments demonstrate that the administration of the immunomagnetic composition and the kit for treating cancer of the present disclosure can change the tumor microenvironment. In this experiment further assesses the change in tumor-specific immune response and systemic effects after administrating the immunomagnetic composition and the kit for treating cancer of the present disclosure. Tumors, sera and spleens of different groups of the 4T1-Luc tumor-bearing mice are collected to analyze the changes of INF-$\gamma^+$CD44$^+$ T cells and CD8$^+$CD3$^+$ T cells. The apoptosis of skin tissue of the 4T1-Luc tumor-bearing mice in different groups are analyzed by a TUNEL assay.

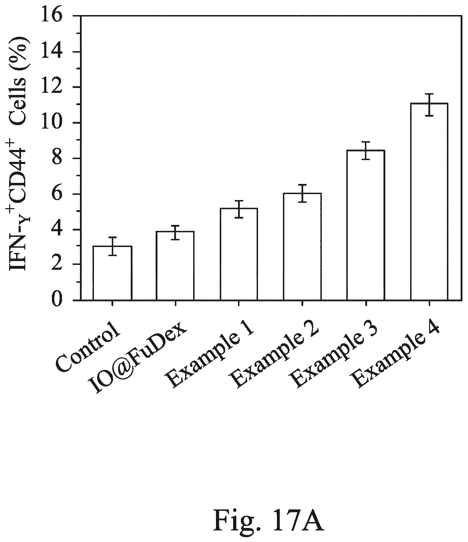

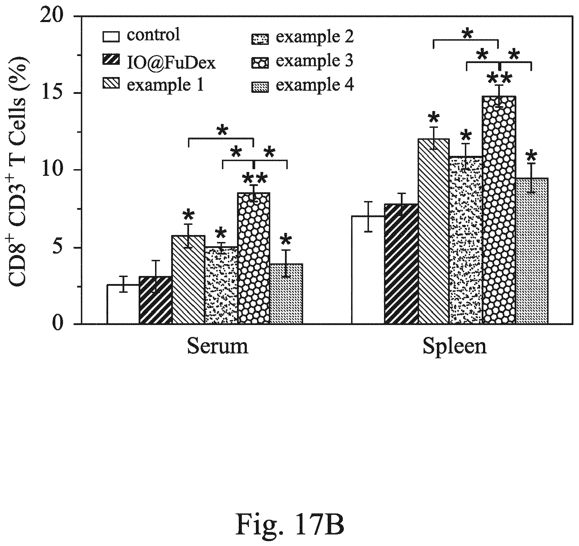

Please refer to Figs. 17A and 17B, which show reaction sites analysis results of the immunomagnetic composition and the kit for treating cancer of the present disclosure. Fig. 17A shows the results of changes in INF-$\gamma^+$CD44$^+$ T cells from different groups of the 4T1-Luc tumor-bearing mice. Fig. 17B shows the results of changes in CD8$^+$CD3$^+$ T cells from different groups of the 4T1-Luc tumor-bearing mice.

In Fig. 17A, the proliferation number of INF-$\gamma^+$CD44$^+$ T cells is increased in the groups administrated with the examples 1-4; in particularly in the group administered with the example 4. In Fig. 17B, the CD8$^+$CD3$^+$ T cells are proliferated in both the serum and the spleen after administrating the examples 1-3. When compared the group administered with the example 4 to the group administered with the example 3, the magnetic navigation reduces the proliferation of the CD8$^+$CD3$^+$ T cells in the serum and the spleen.

Cutaneous toxicity induced pruritus and vitiligo is one of the adverse effects found in the immune checkpoint inhibitor therapy. Since immune-related adverse events are often appear in long-term condition, the TUNEL assay is performed in the skin tissues of the 4T1-Luc tumor-bearing mice at 4 weeks after first dose to evaluate whether these infiltrated T cells induce immune response and cause tissue damage in skin.

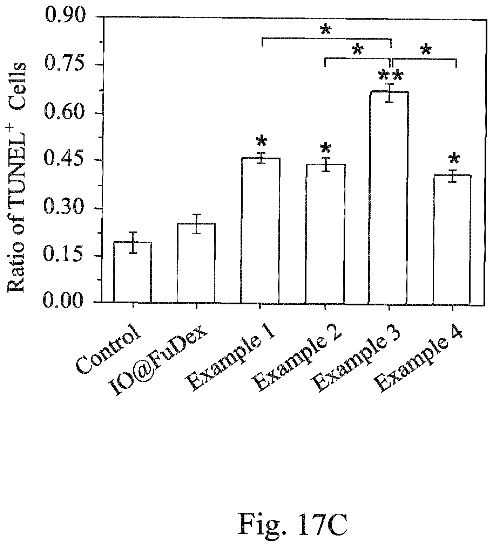

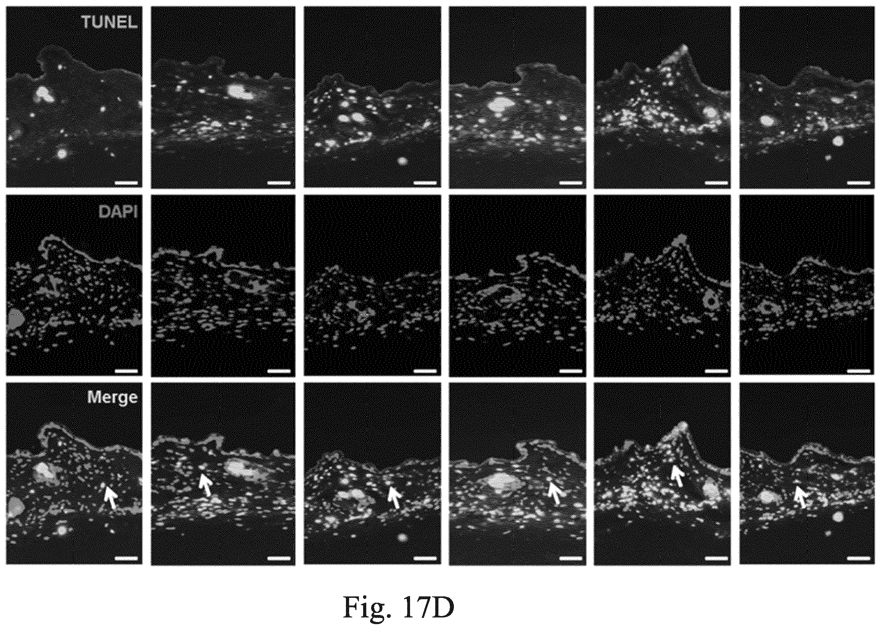

Please refer to Figs. 17C and 17D, which show TUNEL assay results of the immunomagnetic composition and the kit for treating cancer of the present disclosure. Fig. 17C shows the statistical results of the apoptotic index of the skin measured by TUNEL in different groups of the 4T1-Luc tumor-bearing mice, wherein the apoptotic index stands as the ratio of TUNEL$^+$ apoptotic nuclei divided by the total number of nuclei which is counterstained using DAPI from randomly chosen microscopic fields. Differences between groups are evaluated by two-way ANOVA with the Newman-Keuls post hoc test. Fig. 17D shows fluorescent micrographs of the TUNEL assay of the different groups of the 4T1-Luc tumor-bearing mice, where the scale bar represents the length of 50 $\mu$m.

In Figs 17C and 17D, TUNEL-labeled (green) apoptotic cells are increased in the groups administrated with the examples 1-3, wherein the apoptotic index of the group administrated with the example 3 induced a 3.3-fold increase compared to the control group. However, when compared the group administered with the example 4 to the group administered with the example 3, the magnetic navigation effectively reduces the number of apoptotic cells in the skin tissue.

### 2.3 Analysis of immune-related adverse events after administration of the immunomagnetic composition and the kit for treating cancer of the present disclosure

To analyze the safety of the immunomagnetic composition and the kit for treating cancer of the present disclosure, the 4T1-Luc tumor-bearing mice are administrated with the example 3 and the example 4, respectively, in this experiment. The analysis of the Immune-related adverse events (irAEs) in the 4T1-Luc tumor-bearing mice is performed at 4 weeks after the tumor implantation.

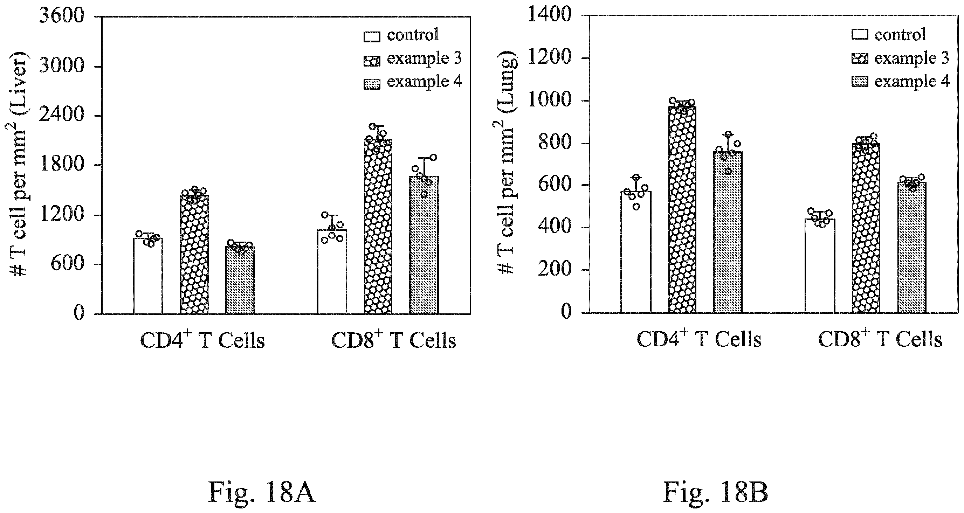

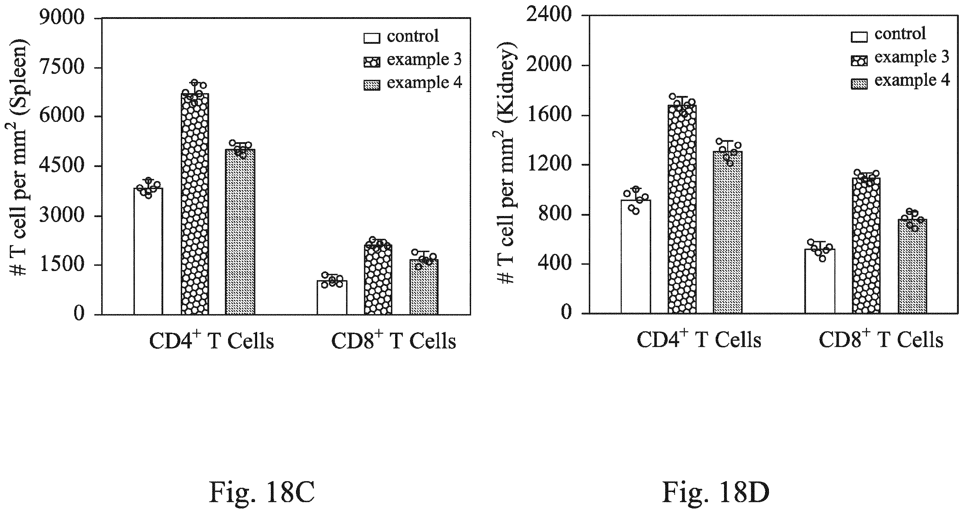

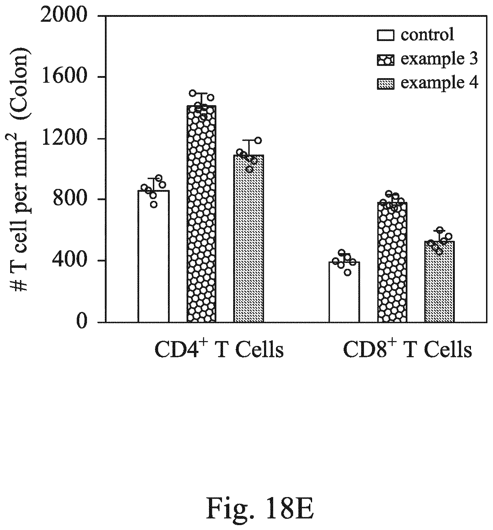

In this experiment, the effect of side effects are determined by observing the infiltration degree of the CD4$^+$ T cells and the CD8$^+$ T cells in major organs of the 4T1-Luc tumor-bearing mice at 4 weeks postadministration of the example 3 or the example 4. Please refer to Figs. 18A to 18E, which are analysis results of infiltration degree of CD4$^+$ T cells and CD8$^+$ T cells of the 4T1-Luc tumor-bearing mice after administering the immunomagnetic composition and the kit for treating cancer of the present disclosure. Fig. 18A shows analysis results of the liver, Fig. 18B shows analysis results of the lung, Fig. 18C shows analysis results of the spleen, Fig. 18D shows analysis results of the kidney, and Fig. 18E shows analysis results of the colon. The results show that the infiltration degree of the T cells of the group administrated with the example 3 in the liver, the lung, the spleen, the kidney and the colon are lower than that of the control group, and the peripheral accumulation (the liver, the lung, the spleen, the kidney and the colon) of the group administrated with the example 4 is more significantly reduced due to the magnetic navigation, thereby reducing the infiltration of the T cells.

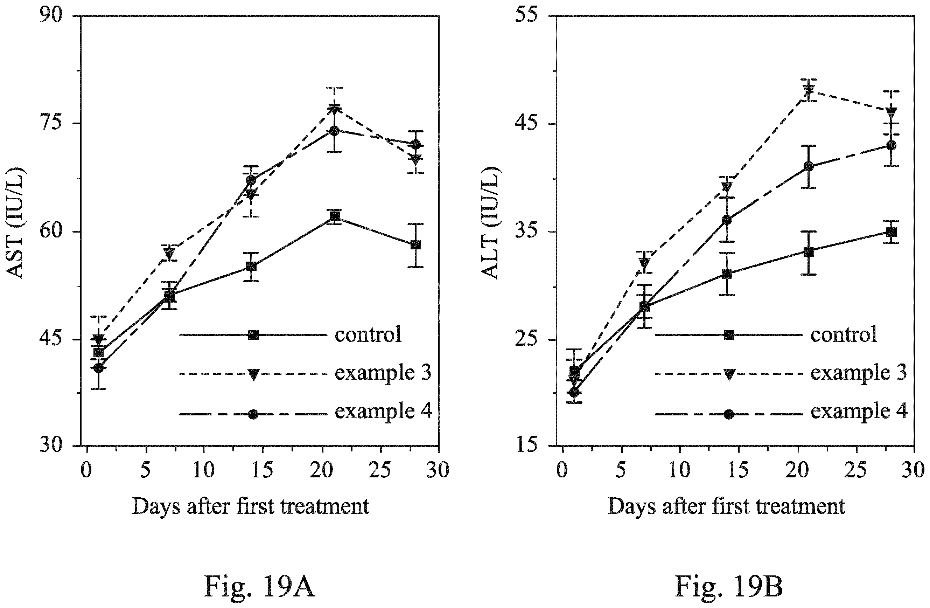

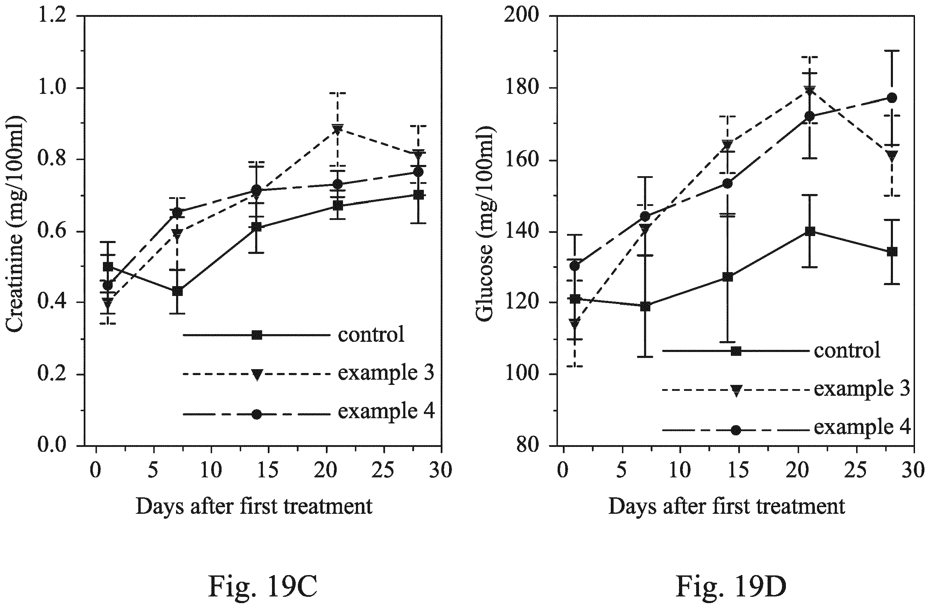

In this experiment, the effect of side effects are further determined by blood biochemical analysis to analyze the liver function index, renal function index, and blood glucose concentration, wherein the liver function index stands as the concentrations of the enzymes aspartate transaminase (AST) and alanine transaminase (ALT), and the renal function index stands as the concentration of the creatinine. Please refer to Figs. 19A to 19D, which are blood biochemical analysis results of the 4T1-Luc tumor-bearing mice after administering the immunomagnetic composition and the kit for treating cancer of the present disclosure. Fig. 19A shows analysis results of AST concentration, Fig. 19B shows analysis results of ALT concentration, Fig. 19C shows analysis results of creatinine concentration, and Fig. 19D shows analysis results of blood glucose concentration. As shown in the Figures, the blood biochemical values of the group administrated with the example 3 or the example 4 are within the normal range as the control group. These results indicate that the immunomagnetic composition and the kit for treating cancer of the present disclosure have biosafety.

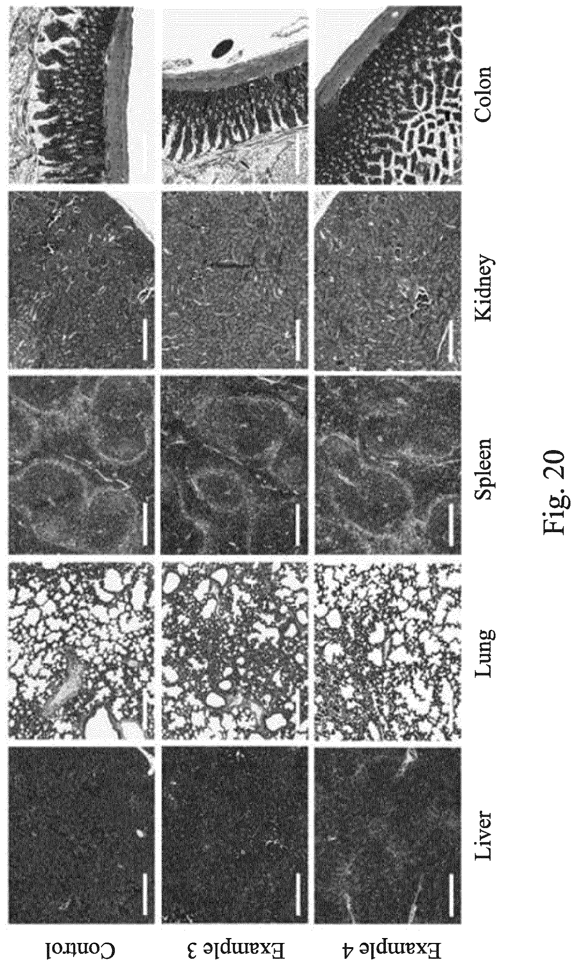

In addition, the histology analysis is performed at 4 weeks after the tumor implantation. Please refer to Fig. 20, which are histology pictures of the 4T1-Luc tumor-bearing mice after administering the example 3 or the example 4. In Fig. 20, compared to the control group, there are no significant tissue damage in the liver, the lung, the spleen, the kidney, and the colon in the 4T1-Luc tumor-bearing mice administrated with the example 3 or the example 4. These results indicate that the immunomagnetic composition and the kit for treating cancer of the present disclosure have biosafety.

To sum up, the fabrication process of the preparation method of the immunomagnetic composition of the present disclosure is simple. In the preparation method of the immunomagnetic composition, the fucoidan with anticancer activity is used as the main component, and combines with the superparamagnetic iron oxide nanoparticles to form the immunomagnetic composition, which can immobilize antibody onto the outer layer and encapsulate the active substance into the core. The fabricated immunomagnetic composition is a nanoscale structure, and its size is suitable to penetrate into the tumor for enhancing the effect of the fucoidan on the tumor. The antibody immobilized onto the outer layer can be the immunosense checkpoint inhibitor and/or the T cell expansion agent, so that the immunomagnetic composition of the present disclosure can also be the immune checkpoint inhibitor and/or the T cell expansion agent in addition to the anti-cancer function of its own material for significantly improving the tumor microenvironment. Moreover, the immunomagnetic composition of the present disclosure can markedly improve the anti-cancer effect of the immunotherapy with the same antibody alone, and can achieve better tumor inhibition with less antibody dosage. The fabricated immunomagnetic composition can be lyophilized to form the powdery crystals and stored under aseptic conditions for a long period of time. The lyophilized powdery crystal can be rapidly re-dispersed in the solvent when needed, showing its convenience and stability.

The kit for treating cancer includes the immunomagnetic composition and the magnetic field generator, wherein the magnetic field generator can be the auxiliary tool to generate the magnetic field for the magnetic navigation. Thus, the immunomagnetic composition of the present disclosure in the kit for treating cancer can be accumulated in the affected part, so that the immune cells can significantly proliferate in the tumor and the immune response of the systemic circulation can be reduce for further enhancing the anti-cancer effect of the immunomagnetic composition of the present disclosure. The results of the aforementioned experiments indicate that the kit for treating cancer has the therapeutic effect of local treatment. Therefore, the kit for treating cancer of the present disclosure simultaneously has the physical target function and biological target function, which is helpful for the immunotherapy with the chemotherapy or the combination therapy of the immunotherapy, and can avoid serious side effects caused by strong immune response.

Although the present disclosure has been described in considerable detail with reference to certain embodiments thereof, other embodiments are possible. Therefore, the spirit and scope of the appended claims should not be limited to the description of the embodiments contained herein. It will be apparent to those skilled in the art that various modifications and variations can be made to the structure of the present disclosure without departing from the scope or spirit of the disclosure. In view of the foregoing, it is intended that the present disclosure cover modifications and variations of this disclosure provided they fall within the scope of the following claims.

**Claims**

1.  An immunomagnetic composition, comprising:

    a core;
    a shell formed by a complex, wherein the complex is fabricated by a combination of a fucoidan, an oxidized dextran, and a plurality of superparamagnetic iron oxide nanoparticles via a hydrophobic interaction, and the core is encapsulated in the shell; and
    an outer layer comprising at least one antibody immobilized onto an outside of the shell to form the outer layer, wherein the antibody is an immune checkpoint inhibitor and/or a T cell expansion antibody.

2.  The immunomagnetic composition of claim 1, wherein the immunomagnetic composition is a sphere with a particle size ranging from 80 nm to 350 nm.

3.  The immunomagnetic composition of claim 1, wherein the fucoidan is extracted from *Undaria pinnatifida*, *Macrocystis pyrifera*, or *Fucus vesiculosus.*

4.  The immunomagnetic composition of claim 1, wherein the oxidized dextran has an aldehyde group.

5.  The immunomagnetic composition of claim 4, wherein the oxidized dextran is prepared from a dextran with a molecular mass ranging from 5 kDa to 270 kDa.

6.  The immunomagnetic composition of claim 1, wherein the immune checkpoint inhibitor is selected from the group consisting of a PD-L1 antibody, a PD-1 antibody, a CTLA-4 antibody and a TIM-3 antibody.

7.  The immunomagnetic composition of claim 1, wherein the T cell expansion antibody is selected from the group consisting of a CD3 antibody, a CD28 antibody and a 4-1BB antibody.

8.  The immunomagnetic composition of claim 1, wherein the core further comprises an active substance.

9.  A preparation method of an immunomagnetic composition, comprising:

    providing an hydrophilic phase solution, wherein the hydrophilic phase solution comprises a fucoidan and an oxidized dextran;
    providing an organic phase solution, wherein the organic phase solution comprises an organic solvent and a plurality of superparamagnetic iron oxide nanoparticles;
    performing an emulsification, wherein the hydrophilic phase solution and the organic phase solution are mixed to form an emulsion;
    removing the organic solvent in the emulsion to form a magnetic fucoidan nanoparticle; and
    performing an antibody immobilization, wherein at least one antibody is mixed with the magnetic fucoidan nanoparticle to form the immunomagnetic composition, and the least one antibody is an immune checkpoint inhibitor and/or a T cell expansion antibody.

10. The preparation method of the immunomagnetic composition of claim 9, wherein the immune checkpoint inhibitor is selected from the group consisting of a PD-L1 antibody, a PD-1 antibody, a CTLA-4 antibody and a TIM-3 antibody.

11. The preparation method of the immunomagnetic composition of claim 9, wherein the T cell expansion antibody is selected from the group consisting of a CD3 antibody, a CD28 antibody and a 4-1BB antibody.

12. The preparation method of the immunomagnetic composition of claim 9, wherein a weight ratio of the fucoidan and the oxidized dextran is 1: 0.1 to 1: 4.

13. The preparation method of the immunomagnetic composition of claim 9, wherein the oxidized dextran has an aldehyde group.

14. The preparation method of the immunomagnetic composition of claim 9, wherein the oxidized dextran is prepared from a dextran with a molecular mass ranging from 5 kDa to 270 kDa.

15. The preparation method of the immunomagnetic composition of claim 9, wherein the organic solvent is methanol, dichloromethane or chloroform.

16. A use of the immunomagnetic composition of any one of claims 1 to 8 in manufacture of a pharmaceutical composition for treating cancer.

17. The use of the immunomagnetic composition of claim 16, wherein the pharmaceutical composition for treating cancer is a pharmaceutical composition for inhibiting a cancer cell proliferation.

18. The use of the immunomagnetic composition of claim 16, wherein the pharmaceutical composition for treating cancer is a pharmaceutical composition for reducing a cancer cell metastasis.

19. The use of the immunomagnetic composition of claim 16, wherein the pharmaceutical composition for treating cancer is a pharmaceutical composition for triggering a tumor immune response.

20. A kit for treating a cancer, comprising:

the immunomagnetic composition of any one of claims 1 to 8; and
a magnetic field generator.

**Fig. 1**

300

```
┌─────────────────────────────────────────────┐
│   Hydrophilic phase solution is provided      │──── 310
└─────────────────────────────────────────────┘
                        ⇩
┌─────────────────────────────────────────────┐
│     Organic phase solution is provided        │──── 320
└─────────────────────────────────────────────┘
                        ⇩
┌─────────────────────────────────────────────┐
│       An emulsification is performed          │──── 330
└─────────────────────────────────────────────┘
                        ⇩
┌─────────────────────────────────────────────┐
│  The organic solvent in the emulsion is removed │──── 340
└─────────────────────────────────────────────┘
                        ⇩
┌─────────────────────────────────────────────┐
│    An antibody immobilization is performed    │──── 350
└─────────────────────────────────────────────┘
```

Fig. 2

Fig. 3A

Fig. 3B

Fig. 3C

Fig. 3D

Fig. 3E

Fig. 3F

Fig. 4C

Fig. 4B

Fig. 4A

Fig. 5A

Fig. 5B

EP 3 636 269 A1

Fig. 6A

Fig. 6C

Fig. 6B

Fig. 7A

Fig. 7B

Fig. 8

Fig. 9

Fig. 10A

Fig. 10B

EP 3 636 269 A1

Fig. 11B

Fig. 11A

Fig. 11C

Fig. 11D

EP 3 636 269 A1

Fig. 11E

Fig. 11F

EP 3 636 269 A1

Fig. 12A

Fig. 12B

Fig. 12C

Fig. 12D

Fig. 13A

Fig. 13B

Fig. 13C

Control     IO@FuDex     Example 1     Example 2     Example 3     Example 4

Fig. 13D

Fig. 14A

Fig. 14B

Fig. 14C

Control    IO@FuDex    Example 1    Example 2    Example 3    Example 4

Fig. 14D

EP 3 636 269 A1

Fig. 14E

Fig. 15A

Fig. 15B

EP 3 636 269 A1

Fig. 15C

Fig. 16A

Fig. 16B

EP 3 636 269 A1

Fig. 16C

Fig. 16D

EP 3 636 269 A1

Fig. 16E

Fig. 16F

EP 3 636 269 A1

Fig. 16G

Fig. 16H

EP 3 636 269 A1

Fig. 16I

Fig. 17A

Fig. 17B

EP 3 636 269 A1

Fig. 17C

Fig. 17D

Fig. 18A

Fig. 18B

EP 3 636 269 A1

Fig. 18C

Fig. 18D

Fig. 18E

Fig. 19A

Fig. 19B

EP 3 636 269 A1

Fig. 19C

Fig. 19D

EP 3 636 269 A1

Fig. 20

EP 3 636 269 A1

# INTERNATIONAL SEARCH REPORT

| International application No. |
|---|
| PCT/CN2018/084769 |

## A. CLASSIFICATION OF SUBJECT MATTER

A61K 31/737 (2006.01) i; A61K 39/395 (2006.01) i; A61P 35/00 (2006.01) i

According to International Patent Classification (IPC) or to both national classification and IPC

## B. FIELDS SEARCHED

Minimum documentation searched (classification system followed by classification symbols)

A61K, ; A61P

Documentation searched other than minimum documentation to the extent that such documents are included in the fields searched

Electronic data base consulted during the international search (name of data base and, where practicable, search terms used)

DWPI; VEN; CNABS; CNTXT; CNKI: 褐藻多糖，褐藻糖，右旋糖酐，磁性，抗体，免疫, fucoidan, dextran, magnetic, checkpoint, inhibit+, immun+

## C. DOCUMENTS CONSIDERED TO BE RELEVANT

| Category* | Citation of document, with indication, where appropriate, of the relevant passages | Relevant to claim No. |
|---|---|---|
| A | CN 104013640 A (LAI, Yuanshu) 03 September 2014 (03.09.2014), claims 1-8 | 1-20 |
| A | CN 102716414 A (DALIAN SHENGHONG MEDICINE CO., LTD.) 10 October 2012 (10.10.2012), claims 1-3 | 1-20 |
| A | US 2012065158 A1 (OKAMOTO YOSHIHARU et al.) 15 March 2012 (15.03.2012), claims 1-15 | 1-20 |
| A | WO 2006091180 A2 (SEZER, ALI DEMIR) 31 August 2006 (31.08.2006), claims 1-20 | 1-20 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 July 2018 | 02 August 2018 |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>WANG, Tao<br><br>Telephone No. (86-10) 62084750 |

Form PCT/ISA /210 (second sheet) (July 2009)

**INTERNATIONAL SEARCH REPORT**
Information on patent family members

| | International application No. |
|---|---|
| | PCT/CN2018/084769 |

| Patent Documents referred in the Report | Publication Date | Patent Family | Publication Date |
|---|---|---|---|
| CN 104013640 B | 28 September 2016 | CN 104013640 A | 03 September 2014 |
| US 2012065158 A1 | 15 March 2012 | WO 2010110223 A1 | 30 September 2010 |
| | | JP WO2010110223 A1 | 27 September 2012 |
| WO 2006091180 A2 | 31 August 2006 | WO 2006091180 A3 | 15 March 2007 |
| CN 102716414 A | 10 October 2012 | None | |

Form PCT/ISA /210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHOUHENG SUN et al.** Monodisperse MFe2O4 (M = Fe, Co, Mn) Nanoparticles. *Journal of the American Chemical Society,* 2004, vol. 126 (1), 273-279